(19) Europäisches Patentamt
European Patent Office
Office européen des brevets

(11) Publication number: **0 423 842 A2**

# (12) EUROPEAN PATENT APPLICATION

(21) Application number: **90122171.3**

(22) Date of filing: **02.08.84**

(51) Int. Cl.$^5$: **C07J 1/00**, A61K 31/565

This application was filed on 20 - 11 - 1990 as a divisional application to the application mentioned under INID code 60.

(30) Priority: **02.08.83 US 519550**

(43) Date of publication of application: **24.04.91 Bulletin 91/17**

(60) Publication number of the earlier application in accordance with Art.76 EPC: **0 133 995**

(84) Designated Contracting States: **BE CH DE FR GB IT LI LU NL**

(71) Applicant: **Research Corporation**
**Suite 853, 25 Broadway**
**New York New York 10174(US)**

(72) Inventor: **Schwartz, Arthur G.**
**220 Locust Street**
**Philadelphia, Pennsylvania(US)**
Inventor: **Williams, John R.**
**824 North Woodbine Avenue**
**Penn Valley, Pennsylvania(US)**

(74) Representative: **Brauns, Hans-Adolf, Dr. rer. nat. et al**
**Hoffmann, Eitle & Partner, Patentanwälte**
**Arabellastrasse 4**
**W-8000 München 81(DE)**

## (54) Steroids and therapeutic compositions containing same.

(57) Steroids of the formula

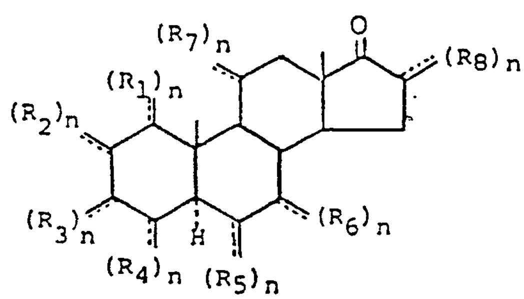

and a therapeutic composition containing same which are useful as anti-cancer, anti-obesity, anti-hyperglycemic, anti-autoimmune and anti-hypercholesterolemic agents.

EP 0 423 842 A2

# STEROIDS AND THERAPEUTIC COMPOSITIONS CONTAINING SAME

The invention described herein was made in the course of work under a grant or award sponsored in part by the National Institutes of Health.

This invention relates to novel steroids and more particularly to androsterone derivatives useful as anti-cancer, anti-obesity, anti-diabetic and hypolipidemic agents.

Dehydroepiandrosterone (DHEA) and DHEA-sulfate are major adrenal secretory products in humans. The plasma concentration of DHEA-sulfate, which, next to cholesterol, is the most abundant steroid in humans, undergoes the most marked age-related decline of any known steroid.

Although, DHEA-sulfate is the main precursor of placental estrogen and may be converted into active androgens in peripheral tissue, there is no obvious biological role for either DHEA or DHEA-sulfate in the normal individual. Several retrospective and prospective studies suggest that women with sub-normal levels of these steroids may be predisposed to develop breast cancer. For example, see Brownsey et al., "Plasma dehydroepiandrosterone sulfate levels in patients with benign and malignant breast disease," Eur. J. Cancer, 8 , 131-137 (1972); Bulbrook et al., "Relation between urinary androgen and corticoid excretion and subsequent breast cancer," Lancet, 2 , 395-398 (1971); Rose et al. "Plasma dehydroepiandrosterone sulfate, androstenedione and cortisol, and urinary free cortisol excretion in breast cancer," Eur. J. Cancer, 13 , 43-47 (1977); Wang et al., "Studies on the sulfate esters of dehydroepiandrosterone and androsterone in the blood of women with breast cancer," Eur. J. Cancer, 10 , 477-482 (1974); and Zumoff et al., "Abnormal 24-hr mean plasma concentrations of dehydroisoandrosterone and dehydroisoandrosterone sulfate in women with primary operable breast Cancer," Cancer Research, 41 , 3360-3363, September 1981.

It has also been established that DHEA is a potent non-competitive inhibitor of mammalian glucose-6-phosphate dehydrogenase (G6PDH). For example, see Oertel et al. "The effects of steroids on glucose-6-phosphate dehydrogenase," J. Steroid Biochem., 3 , 493-496 (1972) and Marks et al. "Inhibition of mammalian glucose-6-phosphate dehydrogenase by steroids," Proc. Nat'l. Acad. Sci, USA, 46 , 447-452 (1960). Moreover, Yen et al. "Prevention of obesity in $A^{vy}$/a mice by dehydroepiandrosterone," Lipids, 12 , 409-413 (1977), reported that long-term administration of DHEA to VY-$A^{vy}$/a mice prevented the development of obesity without suppressing appetite.

Furthermore, it is also known that the long-term treatment of C3H mice with DHEA, in addition to reducing weight gain without suppressing appetite, markedly inhibits spontaneous breast cancer development and may delay the rate of aging. It has been observed that DHEA antagonizes the capacity of the tumor promoter, 12-0-tetradecanoylphorbol-13-acetate, to stimulate $^3$H-thymidine incorporation in mouse epidermis and in a cultured rat kidney epithelial cell line. See, Schwartz, "Inhibition of spontaneous breast cancer formation in female C3H-$A^{vy}$/a mice by long-term treatment with dehydroepiandrosterone," Cancer Res., 39 , 1129-1132 (1979); and Schwartz et al., "Dehydroepiandrosterone: an anti-obesity and anti-carcinogenic agent," Nut. Cancer 3 , 46-53 (1981).

Ben-David et al., "Anti-hypercholesterolemic effect of dehydroepiandrosterone in rats," Proc. Soc. Expt. Biol. Med., 125 , 1136-1140 (1967) have observed that DHEA treatment has an anti-hypercholesterolemic effect in mice, while Coleman et al. (Diabetes 31 , 830, 1982) report that administration of DHEA produces a marked hypoglycemic effect in C57BL/KsJ-db/db mice. The latter authors suggest that the therapeutic effect of DHEA might result from its metabolism to estrogens.

It is further known that DHEA and 16α-bromoepiandrosterone are inhibitors of Eptstein-Barr virus-induced transformation of human lymphocytes and that 16α-bromo-epiandrosterone is a more potent inhibitor of mammalian G6PDH than DHEA. See, Schwartz et al. Carcinogensis, Vol. 2 No. 7, 683-686 (1981).

While DHEA has been found effective in the afore-described manners, there is, however, evidence of an estrogenic effect after prolonged administration. DHEA is not an estrogen *per se* but is well known to be convertible into estrogens. In addition, the therapeutic dose of DHEA is rather high. It would therefore be highly desirable to provide steroids, which while having the same afore-described advantages of DHEA are more potent and do not produce an estrogenic effect.

Accordingly, the present invention provides novel steroids.

The steroids of the present invention exhibit significant and desirable pharmalogical properties, and are particularly useful as cancer preventive agents.

These steroids are additionally useful as anti-obesity agents, anti-hyperglycemic agents, anti-aging agents, and anti-hypercholesterolemic agents.

This invention further provides steroids useful as anti-cancer, anti-obesity, anti-hyperglycemic, anti-aging and anti-hypercholesterolemic agents, which do not evidence estrogenic effects.

The present invention also provides a process for the treatment and/or preventionof cancer, obesity, aging, diabetes, and hyperlipidemia.

The present invention provides novel steroids of the general formula:

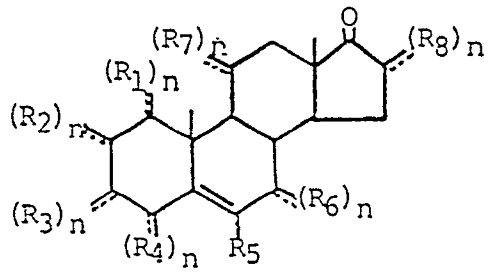

wherein $R_1$, $R_2$, $R_3$, $R_4$, $R_5$, $R_6$, $R_7$ and $R_8$ are each independently selected from the group consisting of hydrogen, alkyl, alkenyl, alkynyl, halogen and hydroxyl, n is an integer from 1 to 2 inclusive with the proviso that when $R_1$, $R_2$, $R_3$, $R_4$, $R_6$, $R_7$ or $R_8$ are alkenyl or alkynyl, n is 1; with the further provisos that when $R_3$ is hydroxy, any one of the substituents $R_1$, $R_2$, $R_4$, $R_5$, $R_6$, $R_7$ or $R_8$ is other than hydrogen; when $R_3$ is hydroxy, $R_1$ may only be alkyl when any one of $R_2$, $R_4$, $R_5$, $R_6$, $R_7$ or $R_8$ is other than hydrogen; when $R_3$ is hydroxy, $R_2$ may only be hydroxy when any one of $R_1$, $R_4$, $R_5$, $R_6$, $R_7$ or $R_8$ is other than hydrogen; when $R_3$ is hydroxy, $R_4$ may only be halogen with $R_1$, $R_2$, $R_5$, $R_6$, $R_7$ or $R_8$ is other than hydrogen; when $R_3$ is hydroxy, $R_6$ may only be hydroxy or methyl when $R_1$, $R_2$, $R_4$, $R_5$, $R_7$ or $R_8$ is other than hydrogen; when $R_3$ is hydroxy, $R_7$ may only be hydroxy when $R_1$, $R_2$, $R_4$, $R_5$, $R_6$ or $R_8$ is other than hydrogen; and when $R_3$ is hydroxy, $R_8$ may only be methyl, ethyl, hydroxy or halogen when $R_1$, $R_2$, $R_4$, $R_5$, $R_6$ or $R_7$ is other than hydrogen; and when $R_3$ is hydroxy, $R_5$ may be alkyl only when $R_1$, $R_2$, $R_4$, $R_6$, $R_7$ or $R_8$ are other than hydrogen. Preferably, $R_3$ is alkyl of from 1 to 10 carbon atoms and most preferably lower alkyl of from 1 to 5 carbon atoms.

The present invention further provides novel steroids of the formula:

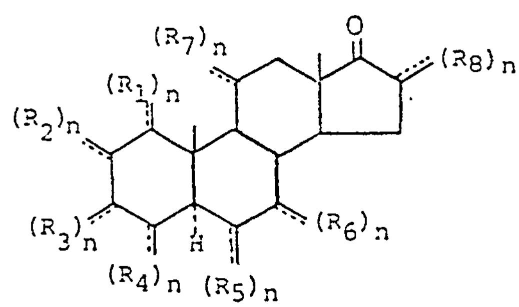

wherein $R_1$-$R_8$ are selected from the group consisting of hydrogen, alkyl, alkenyl, alkynyl, halogen and hydroxyl, n is an integer from 1 to 2 inclusive with the proviso that when $R_1$-$R_8$ are alkenyl or alkynyl n is 1 and with the further provisos that $R_3$ may be hydroxy or halogen only when any one of $R_1$, $R_2$, $R_4$, $R_5$, $R_6$, $R_7$ or $R_8$ is other than hydrogen; when $R_3$ is hydroxy, $R_1$ may be hydroxy or halogen only when any one of $R_2$, $R_4$, $R_5$, $R_6$, $R_7$ or $R_8$ is other than hydrogen; when $R_3$ is hydroxy, $R_2$ may be methyl or halogen only when any one of $R_4$, $R_5$, $R_6$, $R_7$ or $R_8$ is other than hydrogen; when $R_3$ is hydroxy, $R_4$ may be halogen, methyl or hydroxy only when any one of $R_1$, $R_2$, $R_3$, $R_5$, $R_6$, $R_7$ or $R_8$ is other than hydrogen; when $R_3$ is hydroxy, $R_5$ may be methyl, halogen or hydroxy only when $R_1$, $R_2$, $R_4$, $R_6$, $R_7$ or $R_8$ is other than hydrogen; when $R_3$ is hydroxy, $R_6$ may be hydroxy or methyl only when $R_1$, $R_2$, $R_4$, $R_5$, $R_7$ or $R_8$ is other than hydrogen; when $R_3$ is hydroxy, $R_7$ may be hydroxy only when $R_1$, $R_2$, $R_4$, $R_5$, $R_6$ or $R_8$ is other than hydrogen; and when $R_3$ is hydroxy, $R_8$ may be methyl, hydroxy or halogen only when $R_1$, $R_2$, $R_4$, $R_5$, $R_6$ or $R_7$ is other than hydrogen.

The present invention additionally provides processes for the prophylaxis of cancer, obesity, aging, diabetes and hyperlipidemia by administering to a host, e.g. mammals, a therapeutically effective amount of the afore-identified steroids.

The present invention further provides processes for the propyhlaxis of cancer, obesity, aging, diabetes,

hyperlipidemia comprising administering to a host, e.g. mammals, a therapeutically effective amount of the afore-identified steroids or a steroid having the general formula:

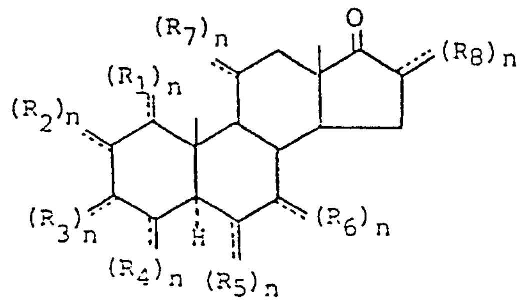

wherein $R_1$-$R_8$ are selected from the group consisting of hydrogen, alkyl, alkenyl, alkynyl, halogen and hydroxyl, n is an integer from 1 to 2 inclusive and with the proviso that when $R_1$-$R_8$ are alkenyl or alkynyl n is 1.

In accordance with the present invention, it has been surprisingly discovered that steroids having a certain structure, described hereinafter in more detail, are characterized with significant pharmacological properties without toxic or undesirable estrogenic effects. That is, it has been quite unexpectedly discovered that the steroids of the present invention are useful as cancer preventive, anti-obesity, anti-diabetic, anti-aging and anti-hypercholesterolemic agents, but unlike DHEA are more potent and exhibit very little or no estrogenic effects.

More particularly, the steroids of the present invention have the general formulas:

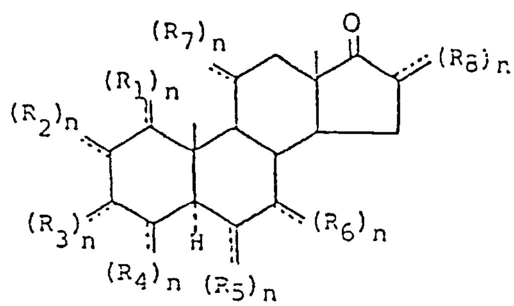

and

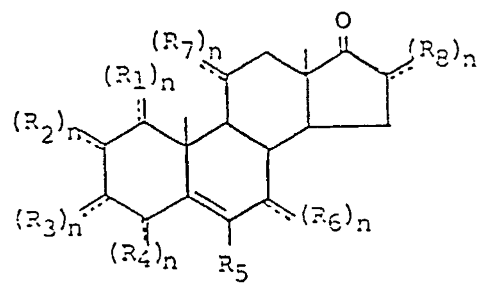

wherein $R_1$, $R_2$, $R_3$, $R_4$, $R_5$, $R_6$, $R_7$ and $R_8$ are as defined hereinbefore. The $R_1$-$R_8$ substituents are designated as being in the $\alpha$-position by means of a broken line (---) joining the substituent to the steroid nucleus, the substituents are designated as being in the $\beta$-position by means of a solid line (—) joining the substituent to the steroid nucleus and in those cases in which the substituent may be either in the $\alpha$- or $\beta$-position the substituents are indicated as being joined to the steroid nucleus by a broken line and a solid line placed side to side. Furthermore, in accordance with I.U.P.A.C. nomenclature, the carbon atoms of the steroids of the present invention are numbered as follows and the steroids have the designated I.U.P.A.C. stereochemistry:

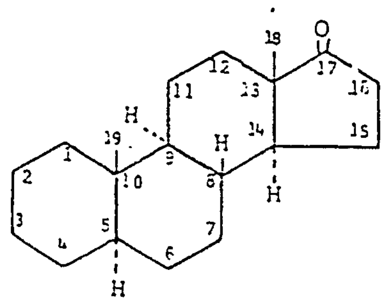

Specific illustrative compounds within the above structural formulas and useful in accordance with the present invention include:

3β-hydroxy-1α-methylandrost-5-en-17-one.
1α-methylandrost-5-en-17-one
1α-methyl-5α-androstan-17-one
3β-hydroxy-2α-methylandrost-5-en-17-one
2α-ethynyl-3β-hydroxyandrost-5-en-17-one
3β-hydroxy-2α,6-dimethylandrost-5-en-17-one
2α,6,16α-trimethylandrost-5-en-17-one
3β-hydroxy-2α,6,16α-trimethylandrost-5-en-17-one
3β-hydroxy-2α-ethynyl-6,16α-dimethylandrost-5-en-17-one
2α-ethynyl-6-chloroandrost-5-en-17-one
3β-methylandrost-5-en-17-one
3β-ethenylandrost-5-en-17-one
3β-ethynylandrost-5-en-17-one
3β-ethynyl-6-methylandrost-5-en-17-one
3β-ethynyl-6-chloroandrost-5-en-17-one
3β-ethynyl-6-chloro-16α-methylandrost-5-en-17-one
3β-ethylandrost-5-en-17-one
3β-butylandrost-5-en-17-one
3β-ethynyl-6,16α-dimethylandrost-5-en-17-one
3β-16α-diethynylandrost-5-en-17-one
3β-ethynyl-6-methyl-16α-ethylandrost-5-en-17-one
3β-ethynyl-7β-methylandrost-5-en-17-one
2α,7β-dimethylandrost-5-en-17-one
1α-chloro-3β-methylandrost-5-en-17-one
3β-hydroxy-4α-methylandrost-5-en-17-one
3β-hydroxy-4α-ethynylandrost-5-en-17-one
3β-hydroxy-4α-ethenylandrost-5-en-17-one
3β-hydroxy-16α-ethyl-4α-ethynylandrost-5-en-17-one
3β-hydroxy-16α-methyl-4α-ethynylandrost-5-en-17-one
2α,3β-dihydroxyandrost-5-en-17-one
2α,3β-diethynylandrost-5-en-17-one
3β-hydroxy-4,6-dimethylandrost-5-en-17-one
3β-methyl-4α-ethynylandrost-5-en-17-one
3β-methyl-7β-chloroandrost-5-en-17-one
3β-methyl-16α-ethylandrost-5-en-17-one
3β-methyl-16α-ethynylandrost-5-en-17-one
3β-hydroxy-6-ethylandrost-5-en-17-one
3β-hydroxy-11α-methylandrost-5-en-17-one
3β-hydroxy-11α-chloroandrost-5-en-17-one
3β-hydroxy-16α-methylandrost-5-en-17-one
3β-hydroxy-16α-ethylandrost-5-en-17-one
3β-hydroxy-16α-ethenylandrost-5-en-17-one
3β-hydroxy-16α-ethynylandrost-5-en-17-one
3β-hydroxy-6-ethenylandrost-5-en-17-one
3β-hydroxy-6-ethynylandrost-5-en-17-one

2α-methyl-3β-hydroxy-6-ethynylandrost-5-en-17-one
3β-hydroxy-7β-methylandrost-5-en-17-one
3β-hydroxy-7β-ethenylandrost-5-en-17-one
3β-hydroxy-7β-ethynylandrost-5-en-17-one
2α-methyl-3β-hydroxy-7β-ethynylandrost-5-en-17-one
2α,3β-dimethylandrost-5-en-17-one
3β,4α-dimethylandrost-5-en-17-one
2α,3β-diethynylandrost-5-en-17-one
3β,4α-diethynylandrost-5-en-17-one
2α,3β-diethenylandrost-5-en-17-one
3β,4α-diethenylandrost-5-en-17-one
2α,3β,6-trimethylandrost-5-en-17-one
3β,4α,7βb-trimethylandrost-5-en-17-one
3β-ethynyl-7β-methylandrost-5-en-17-one
6-methylandrost-5-en-17-one
7β-methylandrost-5-en-17-one
11α-methylandrost-5-en-17-one
16α-methylandrost-5-en-17-one
3β-hydroxy-4,4-difluoroandrost-5-en-17-one
3β-hydroxy-16,16-difluoroandrost-5-en-17-one
2α-fluoro-3β-hydroxyandrost-5-en-17-one
3β-hydroxy-6-bromoandrost-5-en-17-one
3β-methyl-5α-androstan-17-one
3β-methyl-4α-ethynyl-5α-androstan-17-one
3β-methyl-7β-chloro-5α-androstan-17-one
3β-methyl-16α-ethyl-5α-androstan-17-one
3β-methyl-16α-ethynyl-5α-androstan-17-one
3β-hydroxy-6-ethyl-5α-androstan-17-one
3β-hydroxy-11α-methyl-5α-androstan-17-one
3β-hydroxy-11α-chloro-5α-androstan-17-one
3β-hydroxy-16α-methyl-5α-androstant-17-one
3β-hydroxy-16α-ethyl-5α-androstant-17-one
3β-hydroxy-16α-ethynyl-5α-androstan-17-one
3β-hydroxy-6-ethenyl-5α-androstan-17-one
2α-methyl-3β-hydroxy-6-ethynyl-5α-androstan-17-one
3β-hydroxy-7β-methyl-5α-androstan-17-one
3β-hydroxy-7β-ethenyl-5α-androstan-17-one
3β-hydroxy-7β-ethynyl-5α-androstan-17-one
2α-methyl-3β-hydroxy-7β-ethynyl-5α-androstan-17-one
2α,3β-dimethyl-5α-androstan-17-one
3β,4α-dimethyl-5α-androstan-17-one
2α,3β-diethynyl-5α-androstan-17-one
3β,4α-diethynyl-5α-androstan-17-one
2α,3β-diethenyl-5α-androstan-17-one
16α-bromo-3β-methylandrost-5-en-17-one

The steroids of the present invention may be prepared in accordance with conventional organic syntheses or if known may be commercially obtained. The following procedures are illustrative of some procedures which may be utilized to prepare the steroids included herein:

A representative procedure for alkylation at carbon-1 and specifically the synthesis of a 1α-methyl DHEA 3a and 1α-methyl-desoxy DHEA 3b is given in Scheme 1.

Scheme 1

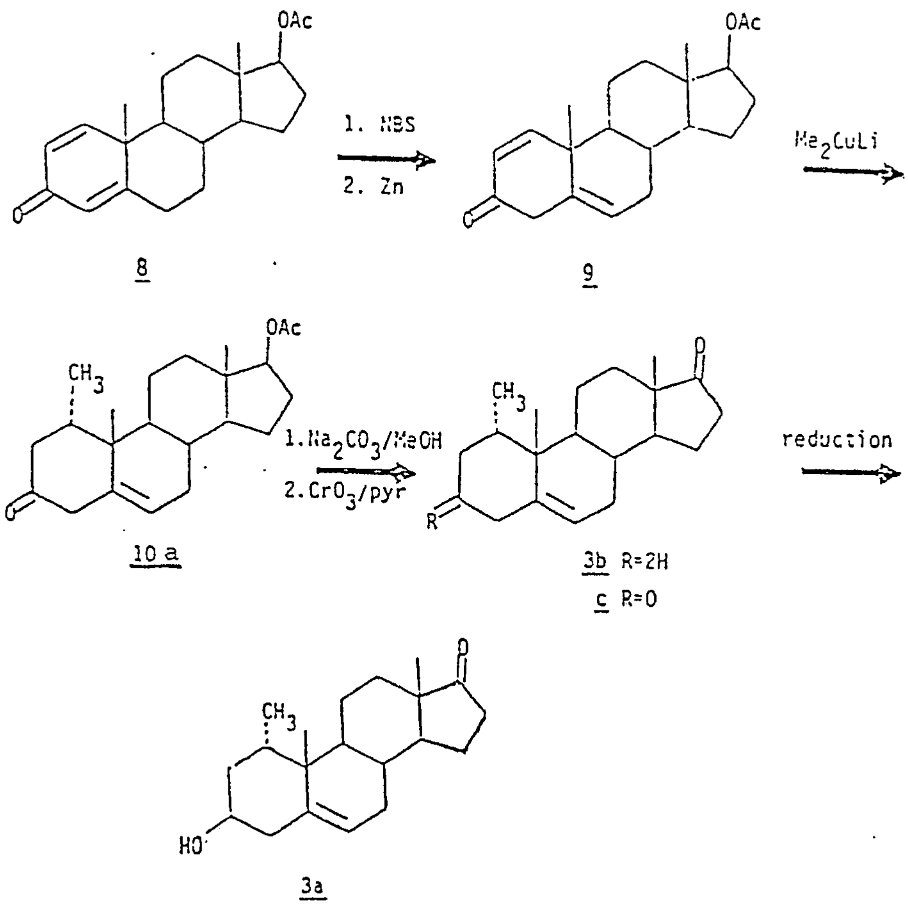


8 9 10 a 3b R=2H c R=O 3a

Allylic bromination (e.g. with N-bromosuccinimide (NBS)) of 17β-acetoxyandrosta-1,4-dien-3-one 8 followed by treatment with zinc affords the non-conjugated enone 9 . 1,4-Alkylation with lithiodimethyl cuprate provides the 1α-methyl ketone 10a . At this stage the 10a may be converted to a methylene by Wolff-Kishner reduction. These vigorous reaction conditions result in hydrolysis of the resulting carbon-17 acetate thereby yielding the hydroxy desoxy derivative, 17β-hydroxy-1α-methylandrost-5-ene (3b) Both 10a and its desoxy derivative can be converted via standard reactions i.e. hydrolysis of the 17-acetate with sodium carbonate and methanol followed by chromium trioxide oxidation of the resulting 17-alcohol to the carbon-17 ketone. Selective reduction of the carbon-3 ketone, 3,17-diketone 3c using sodium borohydride pyridine (pyr) yields Iα-methyl dehydroepiandrosterone 3a .

The following procedures are illustrative for alkylation at carbon-2 and are figuratively illustrated in scheme 2 below.

Methylation of testosterone (1 ) using lithium diisopropylamide (LDA) and methyl iodide afforded a mixture of 2α- and 2β-methyl-17β-hydroxy-4-androsten-3-one (2 & 3 ). Treatment of the mixture with sodium methoxide in methanol epimerizes the 2β-axial methyl to the 2α-configuration (2 ). Acetylation of 2 with acetic anhydride ($Ac_2O$) and p-toluene sulfonic acid (PTSA) in toluene afforded 2α-methyl-3,17β-dihydroxy-3,5-androstadien-3,17-diacetate (4 ). Treatment of the diacetate (4 ) with sodium borohydride in 95% ethanol yielded 2α-methyl-3β,17β-dihydroxy-5-androsten-17-acetate (5 ). Protection of the 3-hydroxy group as a tetrahydropyranyl ether followed by hydrolysis of the 17-acetate yielded 2α-methyl-3β,17β-dihydroxy-5-androsten-3-tetrahydropyranyl ether 7 . Oxidation of the C-17 hydroxy group in 7 followed by hydrolysis of the tetrahydropyranyl ether with hydrochloric acid and aqueous acetone yielded 3β-hydroxy-2α-methylandrost-5-en-17-one (9 ).

The following is a specific example for the synthesis of 2α-methyl DHEA.

To a solution of diisopropylamine (5.3 ml, 38 mmol) in freshly distilled tetrahydrofuran (80 ml) stirred at

-78° C was added n-butyllithium (29.3 ml of 1.3 M in hexane, 38 mmol). This was stirred at -78° C for 30 minutes then warmed to -30° and 17β-hydroxy-4-androsten-3-one (1) (0 g, 17.3 mmol) in tetrahydrofuran (30 ml) was added dropwise. After 30 minutes at -30° C iodomethane (4 ml. 80 mmol) was added. The mixture was allowed to slowly warm to room temperature with stirring, then saturated ammonium chloride solution was added and the product was extracted with ether. The organic layer was dried and the solvent removed to give a mixture of isomers 2 & 3 as an oil (5.26 g) which was used in the next step.

To a stirred solution of sodium (0.75 g, 32 mmol) dissolved in methanol (100 ml) was added the epimeric mixture of 2-methyl-17β-hydroxy-4-androsten-3-one, 2 & 3 (4.93 g, 16.2 mmol) in methanol (100 ml). After 17 hours at room temperature, saturated ammonium chloride solution was added and most of the solvent was removed in vacuo . The product was extracted with dichloromethane, washed with water, dried and the solvent removed to give a gum (4.86 g) which was purified by column chromatography on silica gel. Elution with hexane ether gave 1.6 g of 2 which crystallized from methanol mp 149-151° C;

$H^1$ NMR ($CDCl_3$) δ5.64 (s, 1, H-4), 3.60 (t, 1, H-17, J=9Hz), 1.24 (s, 3, H-19), 1.13 (d, 3, H-2 methyl, J=6 Hz), 0.83 (s, 3, H-18); MS m/e 302($M^+$,33), 260(21), 246(29), 28(100).

A solution of 2α-methyl-17β-hydroxy-4-androsten-3-one (2) (4.86 g, 16.1 mmol) product mixture from previous step in acetic anhydride (40 ml) and paratoluene sulfonic acid (200 mg) in toluene (100 ml) was refluxed 3 1/2 hours. Pyridine (1 ml) was added and the mixture was concentrated on a rotary evaporator then partitioned between methylene chloride and water. The organic layer was dried and the solvent removed. The product mixture (5.78 g) was separated on a flash silica column to give 2α-methyl-3,17β-dihydroxy-3,5-androstadien-3,17-diacetate (4) 1.81 g (27.4%) crystallized from $Et_2O$ - hexane mp 170-171° C.

$H^1$ NMR($CDCl_3$) δ 5.57 (s, 1, H-4), 5.40 (m, 1, H-6), 4.55 (t, 1, H-17, J=9Hz), 2,08 (s, 3, 3-acetate), 2.01 (s, 3, 17-acetate), 1.06 (s, 3, H-19), 0.98 (d, 3, 2 methyl, J=6 Hz), 0,83 (s, 3, H-18); MS m/e 386($M^+$,3) 358(12), 43(100).

A suspension of 2α-methyl-3,17β-dihydroxy-3,5-androstadien-3,17-diacetate (4) (1.31 g, 3.4 mmol) and sodium borohydride (1.3 g) in 95% ethanol (100 ml) was stirred at room temperature for 3 1/2 hours. The solution was cooled to 0° C and glacial acetic acid was added, followed by saturated sodium bicarbonate solution. The product was partitioned between dichloromethane and water, the organic layer dried, and the solvent removed to give 1.23 g product mixture which was separated on 40 g of flash silica column eluted to give 5 , 0.7 g from ether hexane) mp 179-182° C;

$H^1$ NMR ($CDCl_3$) δ 5.27 (m, 1, H-6), 4.62 (t, 1, H-17, J=9Hz), 3.03 (t, 1, H-3, J=9Hz) 2.05 (s, 3, 17-acetate), 1.07 (s, 3, H-19), 1.02 (d, 3, 2-methyl, J=8Hz), 0.83 (s, 3, H-18).

A solution of 2α-methyl-3β,17β-dihydroxy-5-androsten-17-acetate 5 (1.42 g, 4.1 mmol) dihydropyran (DHP) (10 ml) and paratoluene sulfonic acid (100 mg) in ether (50 ml) was stirred at room temperature for 17 hours. The ether solution was washed with saturated sodium bicarbonate solution then water, dried and solvent removed to give the product mixture as an oil (1.65 g) The product was not purified but carried on to the next step.

2α-Methyl-3β,17β-dihydroxy-androst-5-ene-3-tetrahydropyranyl ether 17-acetate, 6 , from the previous step (1.65 g, 3.84 mmol) was dissolved in a solution of 5% potassium carbonate in 4:1 methanol:water (100 ml) and refluxed 1.5 hours. Most of the solvent was removed under reduced pressure and the product was partitioned between chloroform and water. The organic layer was dried and solvent removed to give 1.45 g of the product 7 which was used on the next step.

The product mixture 7 from previous step (1.45 g, 3.84 mmol) was dissolved in pyridine (10 ml) and added to the complex formed by mixing chromium trioxide (2 g) in pyridine (20 ml). This was stirred 2 1/2 hours at room temperature then 1:1 ether-benzene (30 ml) was added and the mixture was filtered through celite then silica gel. The solvent was removed to give the product mixture 8 , 1.52 g as an oil which was carried on to the next step.

A solution consisting of the product mixture 8 from the previous step (1.52 g, 3.94 mmol) and 3N HCl (2 ml) in acetone (40 ml) was stirred at room temperature for 3 hours. Saturated sodium bicarbonate solution was added and the product was extracted with dichloromethane. The organic layer was dried and the solvent removed to give 1.17 g product mixture which was separted on a flash silica column. Elution with 30:70 ether:hexane gave 3β-hydroxy-2α-methyl-androst-5-en-17-one (9) (317 g) which was crystallized from ether:hexane mp 171.5-173;

$H^1$ NMR($CDCl_3$) δ 5.45 (m, 1, H-6), 3.10 (broad m,1, H-3) 1.13 (s, 3, H-19), 1.07 (d, 3, 2 methyl, J=9Hz), 0.97 (s, 3 H-18).

As stated before, the above reactions involving alkylation at carbon-2 are figuratively illustrated in Scheme 2 .

Scheme 2

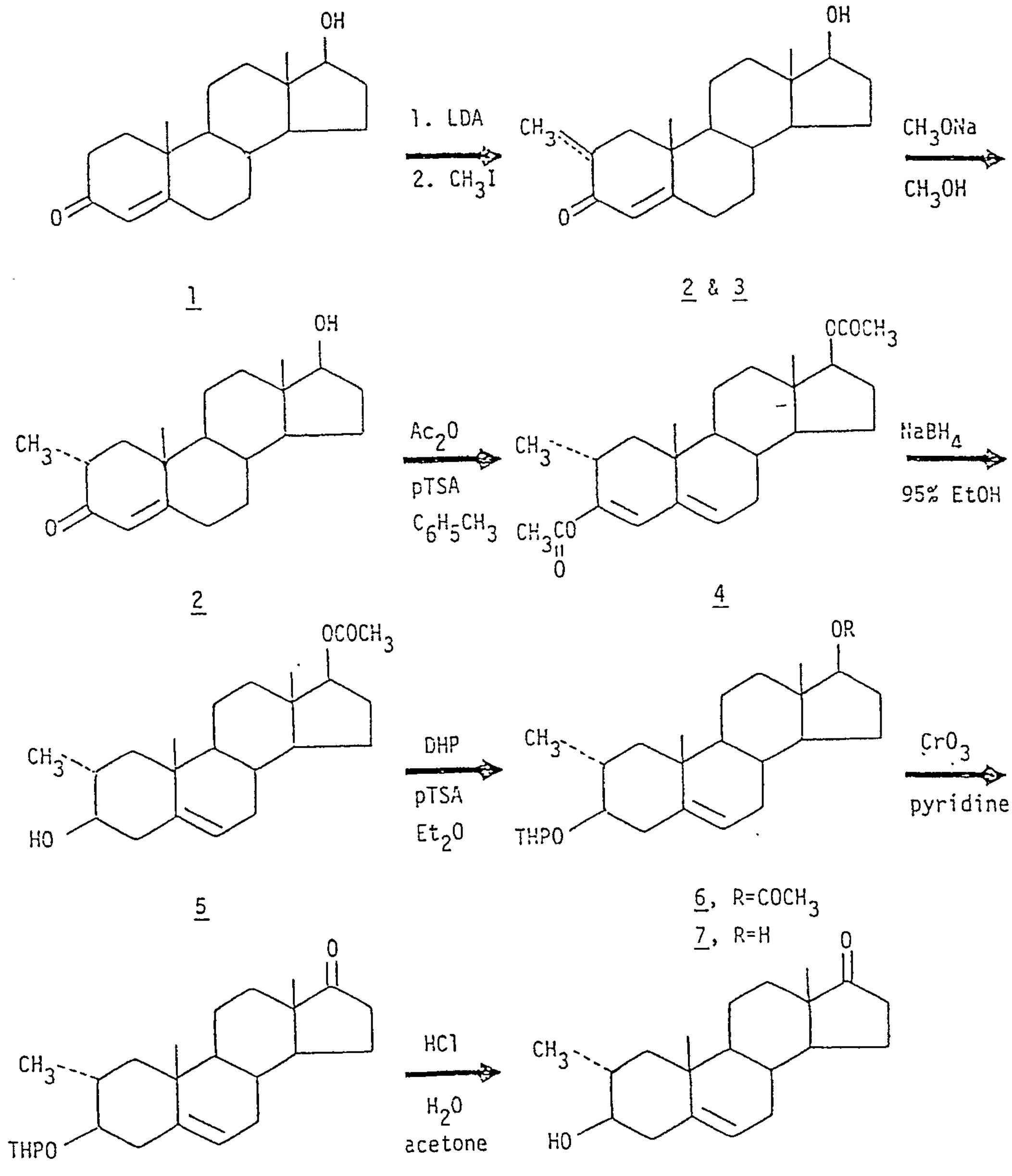


1

2 & 3

2

4

5

6, R=$COCH_3$
7, R=H

8

9

The following procedures are representative for carbon-3 alkylations, shown figuratively in scheme 3 below.

Synthesis of dehydroepiandrosterone with a methyl group replacing the hydroxyl group at carbon-3 is shown below in scheme 3. The methyl configuration at carbon-3 is β, as determined by X-ray analysis. 3β-Hydroxyandrost-5-en-17-one (10) was iodonated at carbon-3 with catechol phosphochloridate followed by iodine. 3β-Iodoandrost-5-en-17-one (11) was ketalized then alkylated with a mixture of methyl lithium and cuprous cyanide, in tetrahydrofuran to yield 3β-methylandrost-5-en-17-ethylene ketal (13). Hydrolysis of the ketal afforded 3β-methylandrost-5-en-17-one (14).

Scheme 3

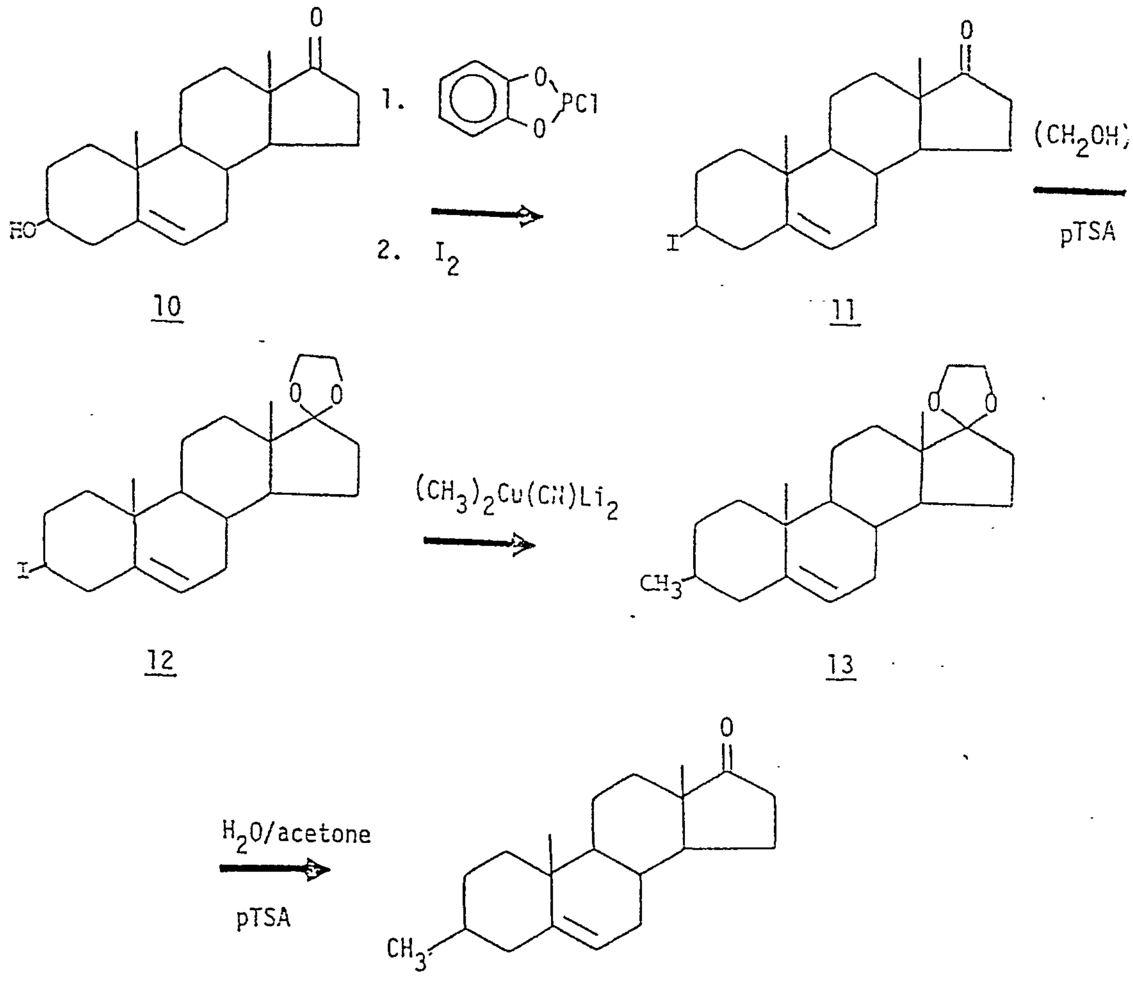


10 11 12 13 14

More specifically, 3β-iodoandrost-5-en-17-one (11) (11.83g, 29.7 mmol) ethylene glycol (20 ml) and p-toluene sulfonic acid (200 mg) in benzene (250 ml) were refluxed under a Dean-Stark trap for 72 hrs. The solution was washed with saturated sodium bicarbonate, water, then dried over magnesium sulfate. Evaporation and recrystallization from ether afforded 11.5g (87.3%) of 3β-iodoandrost-5-en-17-one 17-ethyleneketal (12) :

mp 140-141 °C, IR(KBr) 3010, 2940, 1470, 1425, 1375 $cm^{-1}$

$^1$H NMR ($CDCl_3$) δ 5.44 (brd J=6Hz, 1H, H-6) 3.91 (s, 4H, ketal) 1.07 (s, 3H, C-19 Me) .88 (s, 3H, C-18 Me); MS (m/e) 442 ($M^+$, 1), 380 (35), 315 (57), 253 (67), 227 (11), 105 (24), 99 (100), 91 (35), 55 (27), 41 (33).

Cuprous cyanide (4.465 g, 49.9 mmol) was placed in a dry 500 ml 3 neck round bottom flask equipped with a magnetic stirrer. The system was flushed with $N_2$ and dry THF (30 ml) was added. The suspension was cooled to -78 °C and MeLi 1.5 M (66.5 ml, 99.8 mmol) was added via syringe. The solution was allowed to warm to 0 °C for 5 min., which resulted in a clear tan solution.

After recooling to -78 °C, the 3β-iodo-17-ketal (3) (7.35 g 16.6 mmol) in 40 ml dry tetrahydrofuran was added via a syringe and the solution allowed to warm to room temperature and stirred for 18 hrs. under $N_2$. The solution was extracted with 100 ml of 90% saturated $NH_4Cl$/10% conc. $NH_4OH$. The organic layer was separated, dried over $MgSO_4$ and evaporated to give 6.69 g of crude product. Chromatography on flash silica (240 g) and elution with 1% $Et_2O$/99% hexane gave 6.41 g of colorless crystals. Recrystallization from methanol (200 ml) gave 3β-methylandrost-5-en-17-one 17-ethyleneketal (4). mp 121-122 °C

| | | |
|---|---|---|
| Anal. Calc. | C 80.06 | H 10.38. |
| Found | C 80.12 | H 10.55 |

IR(KBr) 3010, 2930, 1450, 1430, 1370; $^1$H NMR ($CDCl_3$) δ 5.33 (brd J=6Hz), 1H, H-6) 3.90 (s, 4H, ketal) 1.03 (s, 3H, C-19 Me) .91 (s, 3H, C-18 Me) .97 (d, 3H, C-3 Me); MS (m/e) 330 ($M^+$, 16), 316(7), 268(29), 253(22), 239(9), 99 (100), 91(22), 55(27), 41(22).

The 3β-methylandrost-5-en-17-one 17-ethyleneketal (13) (2.20 g 6.7 mmol) was dissolved in acetone (100 ml). p-Toluene sulfonic acid (100 mg) and $H_2O$ (20 ml) were added and the solution refluxed for 2 hrs. The solution was evaporated, taken up in ether (30 ml), washed with saturated $NaHCO_3$, $H_2O$, then dried over $MgSO_4$. The solution was filtered and evaporated to give a colorless solid which was recrystallized from methanol to give 3β-methylandrost-5-en-17-one (14) colorless plates 1.17 g (61%).

mp 148-150° C; IR(KBr) 3010, 3910, 1740, 1455, 1430, 1365;

H$^1$NMR ($CDCl_3$) δ5.41 (brd, J=6Hz, 1H, H-6) 1.11 (s, 3H, C-19 Me) 0.99 (s, 3H, C-18 Me) 1.07 (d, 3H, C-3 Me); MS (m/e) 286 ($M^+$, 58) 271(51), 229 (31), 159 (36), 105 (72), 91 (95), 79 (89), 55 (9), 41 (100).

| | | |
|---|---|---|
| Anal. Calc. | C 83.85 | H10.55 |
| Found | C 83.66 | H10.65 |

Androst-5-en-17-one 15 (Desoxy DHEA)

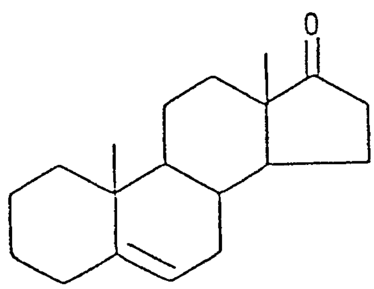

15

Androst-5-en-17-one (15) mp 106° is synthesized in accordance with T. Nambara and H. Takahaski, Chem. Pharm. Bull. Jap., 1970, 18, 2309 m.p. 108-109° C.

A procedure for carbon-4 alkylation and the synthesis of 4α-methyl DHEA is given in Scheme 2.

Scheme 4

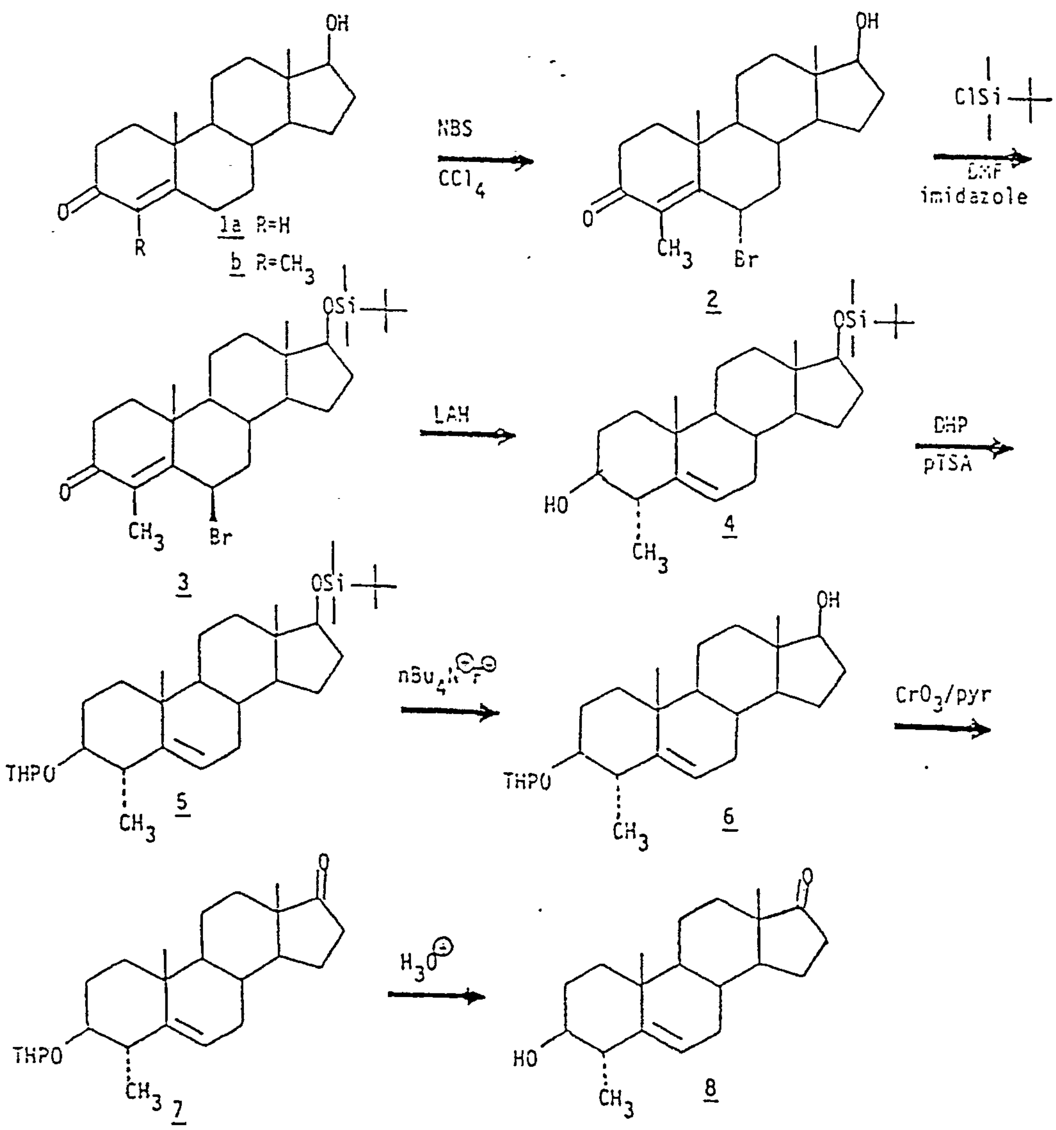


With reference to Scheme 4, alkylation of testerone 1a using potassium t-butoxide and methyl iodide according to the method of Atwater yielded 4-methyltestosterone 1b . Allylic bromination of 4-methyltestosterone using N-bromosuccinimide in carbon tetrachloride yields the 6β-bromo-4-methylandrost-4-en-17β-ol-3-one 2 .Protection of the C-17 alcohol as its t-butyldimethyl silyl derivative yields 3 . Lithium aluminum hydride reduction of the ketone in 3 with concomitant double bond migration and loss of bromide should yield 4 . Protection of the C-3 alcohol as a tetrahydropyranyl ether, followed by deprotection and oxidation of the C-17 alcohol should yield the C-17 ketone 7 . Removal of the C-3 tetrahydropyranyl ether should yield 4α-methyl dehydroepiandrosterone 8 .

ALKENYLATION AND ALKYLATION AT CARBON-6

Steroids may be alkylated at carbon-6 using the method of U. Stache and W. Fritsch Liebigs Analen 1966, 697 , 204.

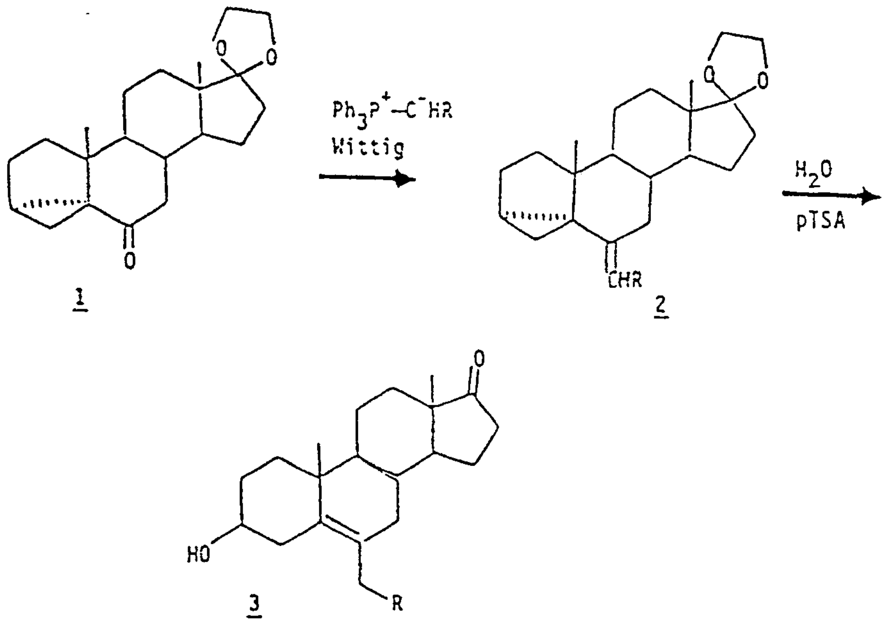


1 2 3

3α,5-Cyclosteroids such as 3α,5-cyclo-5α-androstan-6,17-dione 17 ketal 1 are readily available by solvolysis of steroidal 5-ene-3β-tosylates and mesylates followed by oxidation of the C-6 hydroxyl group, Methylenation of 1 affords 6-methylene-3α,5-cyclo-5α-androstan-17-one 17-ketal 2 (R = H). Treatment of 2 with aqueous acid results in the addition of water and the formation of 3β-hydroxy-6-methylandrost-5-en-17-one, 3 (R = H). Alkenylated derivatives of 3 may be synthesized starting with the appropriated substituted Wittig reagent, such as

$$Ph_3P^{\oplus}-\overset{\ominus}{C}H-CH=CH_2.$$

Alkylation at C-7

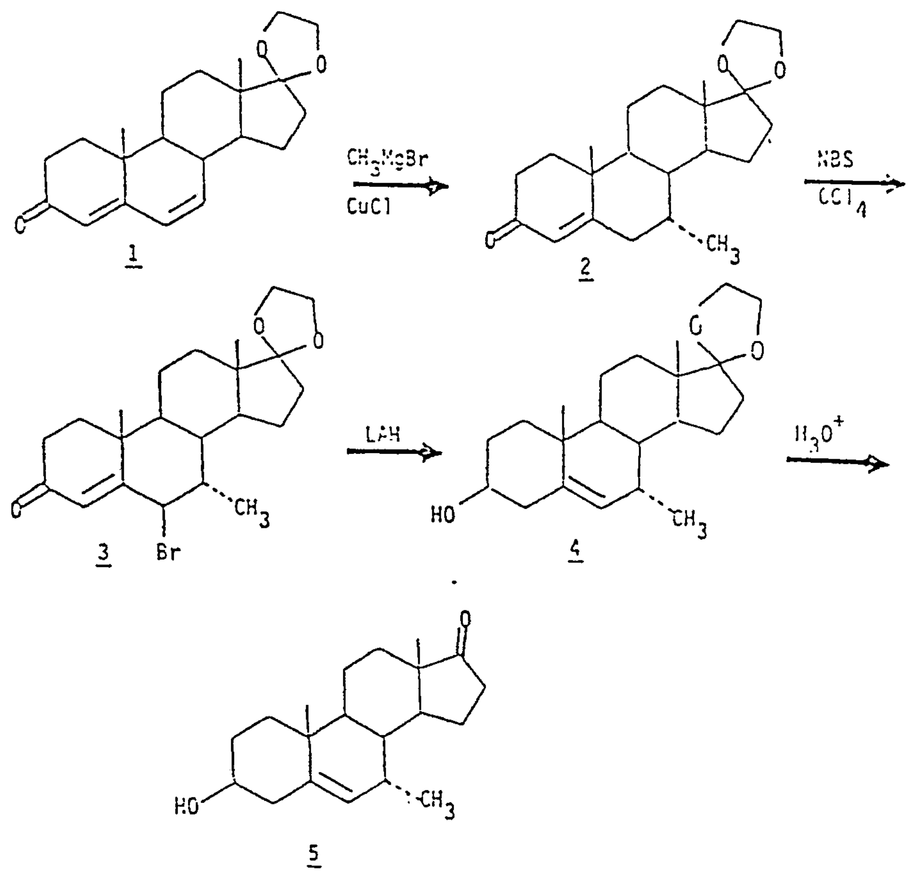


Alkylation of androsta-4,6-dien-3,17-dione 17 ketal 1 with methyl magnesium bromide in the presence of cuprous chloride, proceeds via conjugate addition to yield 7α-methylandrost-5-en-3,17-dione 17 ketal 2 . Allylic bromination of 2 using N-bromosuccinimide in carbon tetrachloride yields the 6β-bromo-7α-methylandrost-4-en-3,17-dione 17 ketal 3 . Lithium aluminum hydride reduction of the ketone in 3 with concomitant double bond migration and loss of bromide should yield 4 . Deprotection of the C-17 ketone with aqueous acid yields 3β-hydroxy-7α-methylandrost-5-en-17-one, 5 . Higher homologues may be synthesized using the substituted Grignard reagent i.e. R = $CH_3$, $C_2H_5$, $C_3H_7$. The 7β-epimer can be synthesized by treatment of 2 with DDQ--dichlorodicyanoquinone to generate another olefin at C-7. Catalytic reduction of this olefin should occur from the α face of the steroid to yield the 7β-methyl steroid i.e. 7β-methylandrost-5-en-3,17-dione 17 ketal. Following the same sequence as above yields 3β-hydroxy-7β-methylandrost-5-en-17-one.

Alkylation at Carbon-11

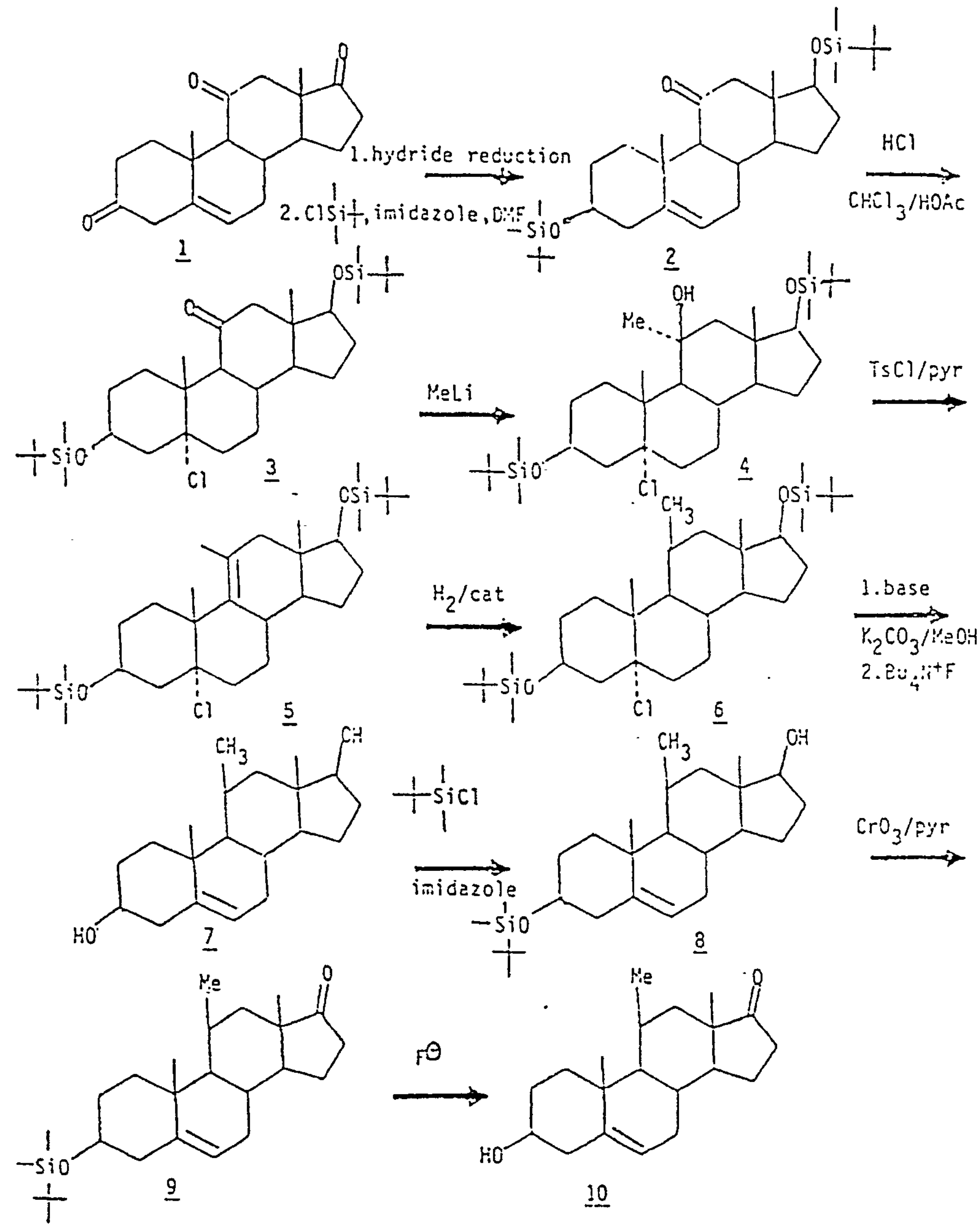


Due to the hindered nature of the C-11 ketone, selective reduction of androst-5-en-3,11,17-trione 1 with hydride should yield the C-3, C-17 dihydroxy steroid 2a , R = H which is protected as its bis(dimethyl-tert-butylsilyl)ether 2b R = $Si(CH_3)_2$t-Bu. Addition of hydrogen chloride across the C-5 olefin affords 5α-chloro-3β,17β-dihydroxyandrost-5-en-11-one 3,17-bis(dimethyl-t-butylsilyl) ether 3 . Alkylation with methyl lithium proceeds from the less hindered α face to yield 5α-chloro-11α-methylandrostan-3β,11β,17β-triol-3,17-bis-(dimethyl-t-butylsilyl) ether 6 . Treatment of the chloro silyl ether 6 with base followed by tetrabutyl ammonium fluoride affords 11β-methylandrost-5-en-3β,17β-diol 7 . Selective silylation yields 11β-methylandrost-5-en-3β,17β-diol 3-dimethyl t-butylsilyl ether 8 . Oxidation of the C-17 alcohol in 8 yields 9 and deprotection of the 3-alcohol yields 11β-methylandrost-5-en-3β-ol-17-one 10 . (11β-methyl DHEA).

Alkylation at Carbon-16

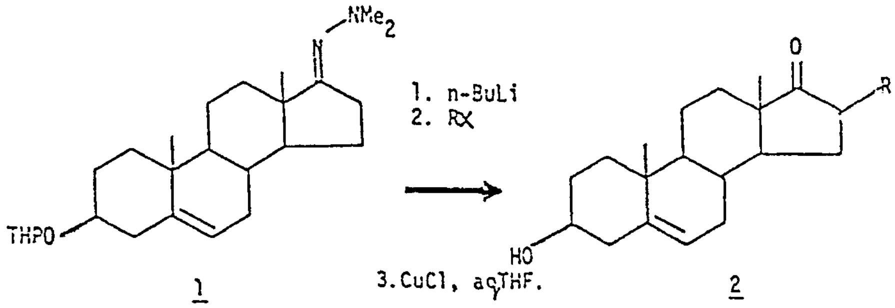


1 3.CuCl, aqTHF. 2

Alkylation of the 17-ketodimethylhydrazone of DHEA 3-tetrahydropyranyl ether using n-butyl lithium as the base followed by an alkyl halide RX, afforded the 16α-alkylated steroid. Hydrazone cleavage with cuprous chloride in aqueous tetrahydrofuran led to regeneration of the C-17 ketone and concomitant cleavage of the tetrahydropyranyl ether resulting in the 16α-alkyl-3β-hydroxy-androst-5-en-17-one 2 . Similarly, 3-β, 16α-dimethyl-androst-5-en-17-one may be prepared by alkylation of 3-β, methyl androst-5-en-17-one using this procedure to introduce the 16α-methyl group, as illustrated by the following procedure:

Diisopropyl amine (1.165g, 11.5mmol) was dissolved in dry tetrahydrofuran (30ml) at-78° C under $N_3$. n-Butyl lithium (4.44ml of a 2.6M solution in hexane, 11.5 mmol) was added via syringe and the solution warmed to -23° C ($CO_2$, $CCl_4$) for .25h. 3β-methyl androst-5-en-17-one (3.0g, 10.4 mmol) in dry tetrahydrofuran (30ml) was added via syringe and the solution stirred for .25h. Methyl iodide (7.0g, 49.33 mmol) in dry tetrahydrofuran (30ml) was added dropwise and the mixture stirred at room temperature for 1.5 h. The solution was quenched with saturated ammonium chloride and the organic layer separated, dried over magnesium sulfate, filtered and evaporated. The residual solid was chromatographed on flash silica gel (120g) and eluted with 1/99 (viv) ether hexane to give 3β,16α-dimethyl-androst-5-ene-17-one (2.3g, 74%) M.P. 109-110° C (recrystallized from methanol). NMR($CDCl_3$)d 5.29(brd, J=5Hz, 1H, H-6), 2.52(m, 1-H, H-16) 1.07 (d J=8Hz, 3H, C-16Me), .99 (S, 3H, C-19Me), .91(S, 3H, C-18Me). IR(kBr) 2900, 1730, 1450, 1430, 1370. Anal. calc. for $C_{21}H_{32}O$,C-83.93, H-10.73. Found C-83.79, H-10.52. MS 300M+(100) 285(62), 282(2), 272(12), 267(17), 229(20), 217(30), 159(17).

The following procedures are illustrative of alkenylation and alkynylation at Carbon-1.

Alkenylation (-CH = CHR) may be effected using the vinyl analogue of the organocuprate reagent i.e. $(CHR\text{-}CH)_2$ CuLi as in Scheme 1 above. Alkynylation (-C≡C-R) using dialkynyl lithium cuprate is possible but this reagent is extremely sluggish. However, using a tri-n-butylstannyl ethylene which may be oxidized by lead tetraacetate to an acetylene (E. J. Corey and R. H. Wollenberg, J. Amer. Chem. Soc. , 1974, 96 , 5581) affords a convenient method for the introduction of an acetylide group. Thus using 2-tri-n-butylstannyl ethenyl 1′-pentynyl lithium cuprate ([$C_3H_7$C≡C-Cu-CH=CHSn $nBu_3$]Li), tri-n-butylstannylethylene is added to the steroid. Oxidation using lead tetraacetate proceeds with the loss of tin and affords the corresponding acetylide. Also aluminum acetylides undergo conjugate addition to enones.

Alkenylation and Alkynylation at Carbon-2

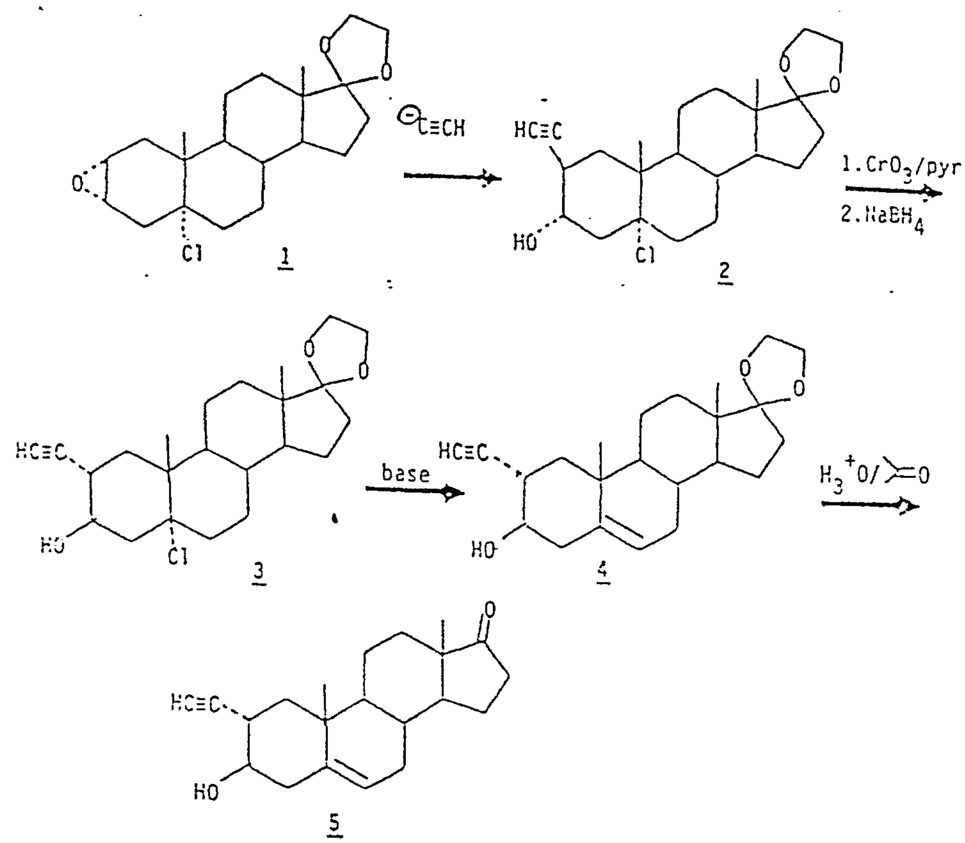


1 2 3 4 5

Reaction of 5α-chloro-2α,3α-epoxyandrostan-17-one 17 ketal 1 with lithium acetylide ethylene diamine complex yields 5α-chloro-2β-ethynylandrostan-3α-ol-17-one 17-ketal 2 . Epimerisation of the C-3 alcohol by oxidation to the C-3 ketone (chromium trioxide/pyridine) and reduction with sodium borohydride affords 5α-chloro-2β-ethynylandrostan-3β-ol-17-one 17-ketal 3 . Deprotection of the C-5 olefin and 17-ketone by treatment first with base ($K_2CO_3$ in methanol) followed by aqueous acid yields 2α-ethynyl-3β-hydroxyandrost-5-en-17-one 5 . The 2α-ethenyl steroid can be synthesized from the ethynyl derivative by careful catalytic reduction with Lindlar catalyst to yield 2α-ethenyl-3β-hydroxyandrost-5-en-17-one.

Alkenylation and Alkynylation at Carbon-3

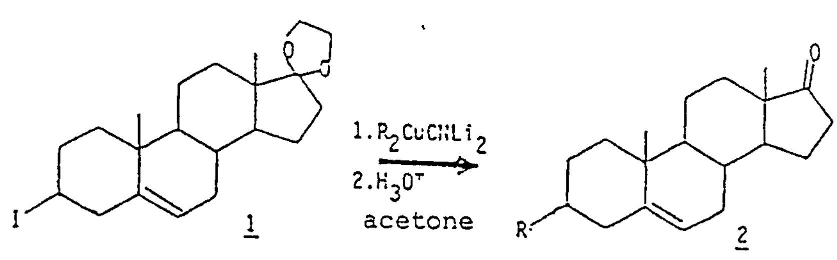


1 2

Reaction of 3β-iodoandrost-5-en-17-one 17-ketal 1 with the organo cuprate reagent $(R^1CH=CH)_2$ Cu-(C≡N)$Li_2$ generated from the appropriate vinyl lithium reagent and cuprous cyanide, should yield the 3β-alkenylandrost-5-en-17-one 2 (R = CH = CHR$^1$) following removal of the C-17-ketal. Similarly, reaction of 1 with the tri-n-butyl stannyl derivative $R^1$ = $nBu_3Sn$ yields upon oxidation with lead tetraacetate and hydrolysis of the C-17 ketal, 3β-ethynylandrost-5-en-17-one 2 ( R = C≡CH).

Alkenylation and Alkynylation at Carbon-4

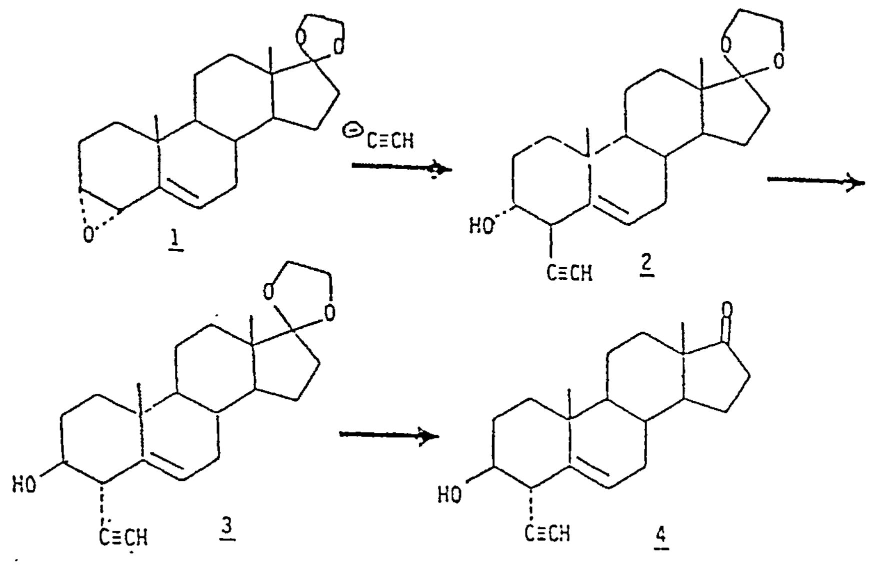


Reaction of 3α,4α••epoxyandrost-5-en-17-one-17-ketal 1 with lithium acetylide diethylamine complex affords 4β-ethynyl-3α-hydroxyandrost-5-en-17-one 17-ketal 2. Epimerisation of the C-3 alcohol by oxidation to the C-3 ketone with chromium trioxide/pyridine followed by reduction with sodium borohydride affords 4β-ethynyl-3, β-hydroxyandrost-5-en-17-one 17-ketal 3 . Careful hydrolysis of the C-17 ketal affords 4α-ethynyl 3β-hydroxyandrost-5-en-17-one, 4 .

Alkynylation at Carbon-6

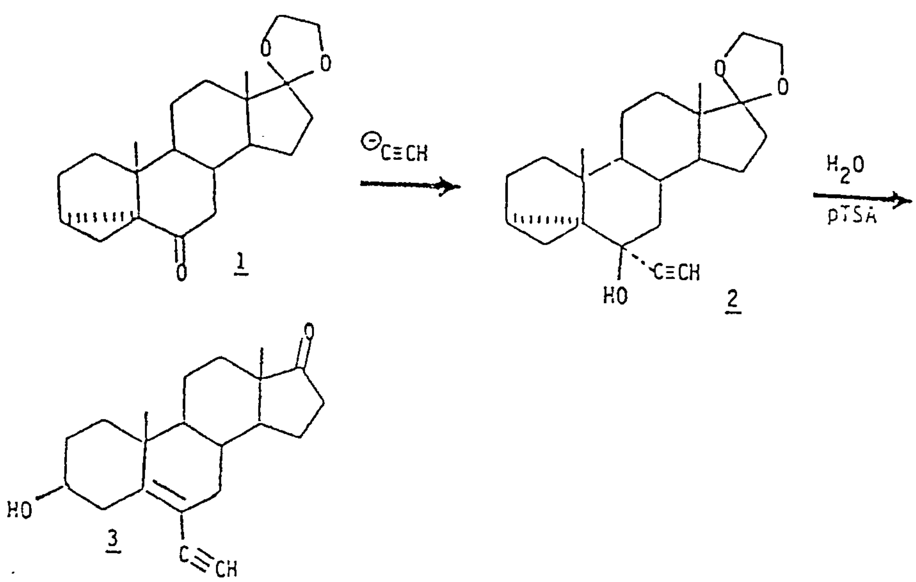


Treatment of 3α,5-cyclo-5-androstan-6,17-dione 17-ketal 1 , with lithium acetylide diethyl amine complex yields 6α-ethynyl -6β-hydroxy-3α,5-cyclo-5α-androstan-17-one 17-ketal 2 . Reaction of 2 with aqueous acid yields 6-ethynyl-3β-hydroxyandrost-5-en-17-one, 3 .

Alkenylation and Alkynylation at Carbon-7

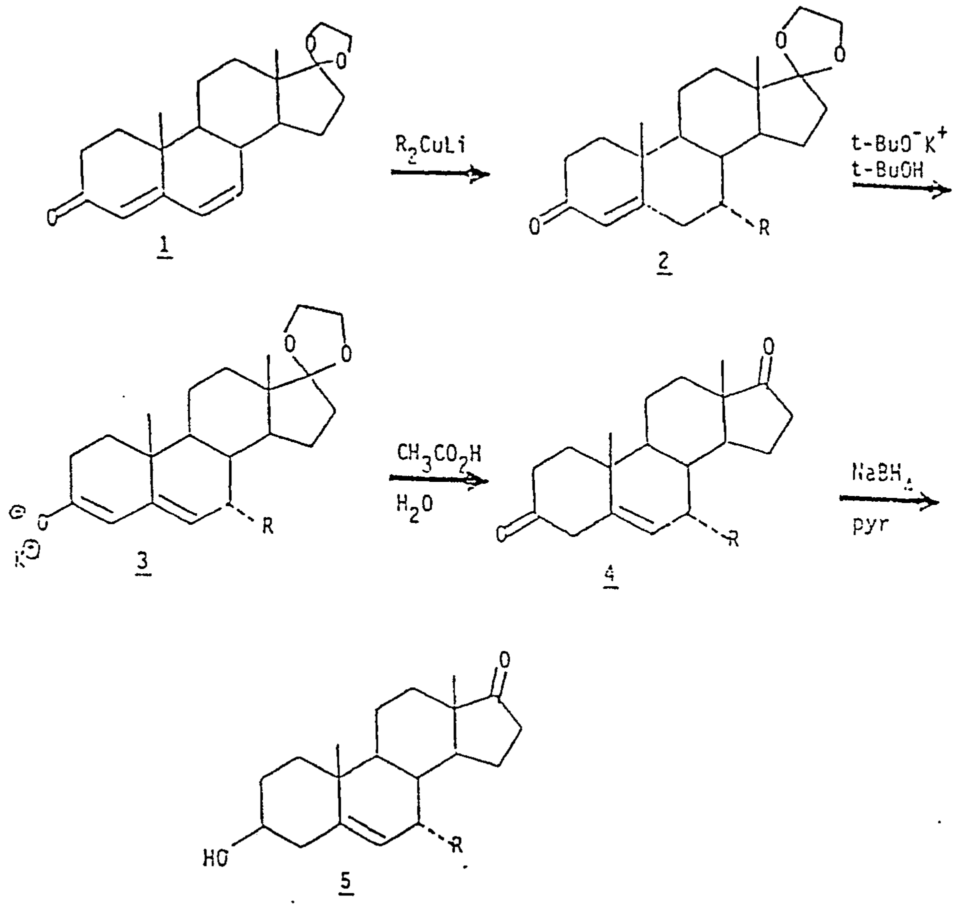


1 2 3 4 5

Alkenylation and alkynylation of androsta-4,6-dien-3,17-dione 17-ketal 1 with $(CHR=CH)_2$ CuLi yields the 7α-alkenyl steroid 2 . Treatment of 2 with potassium t-butoxide in t-butanol yields the dienolate 3 which upon protonation with acetic acid yields 7α-alkenylandrost-5-en-3,17-dione 4 . Selective reduction of the C-3 ketone using sodium borohydride in pyridine yields 3β-hydroxy-7α-alkenyl-androst-5-en-17-one, 5 ($R=CH=CHR^1$). Alkynylation may be effected using 2-tri-n-butylstannyl ethenyl 1′-pentynyl lithium cuprate ($[C_3H_7C \equiv C\text{-}Cu\text{-}CH=CHSnBu_3]Li$), as the alkynylating reagent. The tri-n-butylstannylethylene added by this reagent is oxidized using lead tetraacetate resulting in the loss of tin and the formation of an acetylide, namely 3β-hydroxy-7α-alkynylandrost-5-en-17-one, 5 ($R\text{-}C \equiv CH$).

Alkenylation and Alkynylation at Carbon-11

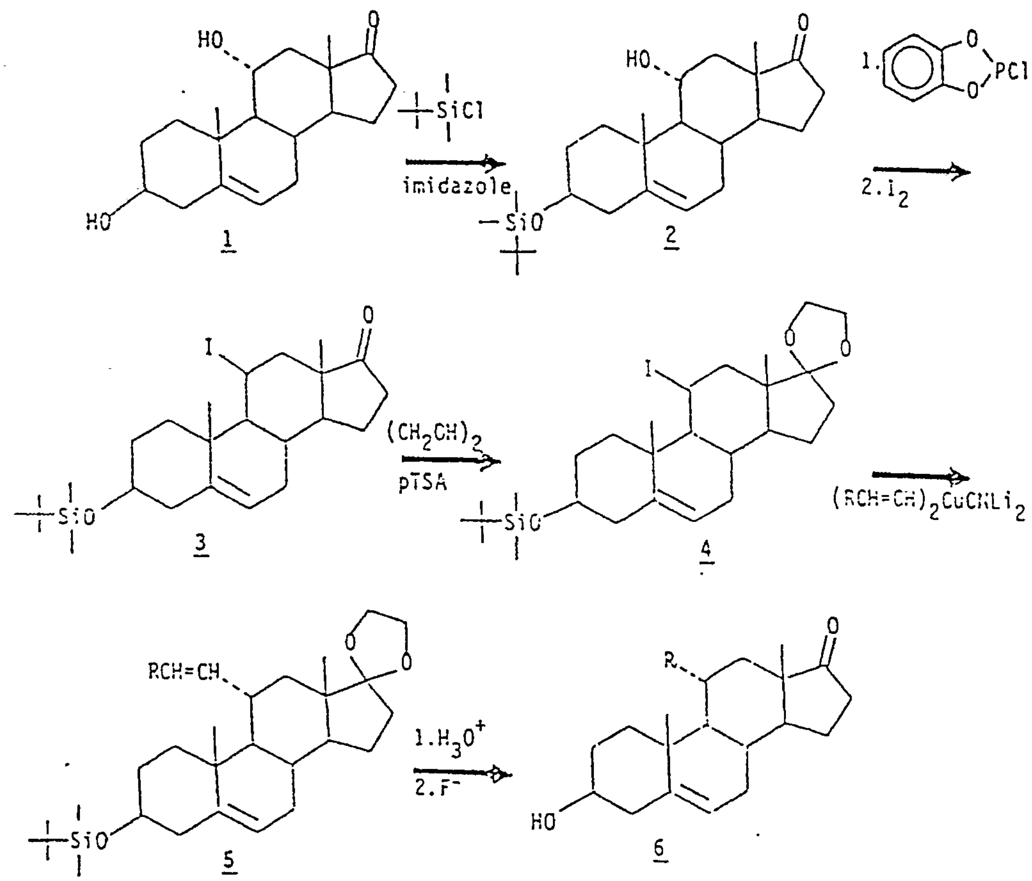


Reaction of the less hindered 3β-hydroxyandrost-5-en-17-one 1 with t-butyldimethylsilyl chloride yields the 3β-t-butyldimethylsilyl ether 2 . Treatment of this first with catechol phosphochloridate followed by displacement with iodine yields 3β-hydroxy-11β-iodoandrost-5-en-17-one dimethyl-t-butylsilyl ether 3 . Protection of the C-17 ketone as the 1,3-dioxolane 4 followed by alkenylation using dialkenyl dilithio cyano cuprate, $(RCH=CH)_2$ $CuCNLi_2$ yields 11α-alkenyl-3β-hydroxyandrost-5-en-17-one t-butyldimethylsilyl ether 5 . Deprotection of the C-17 ketone and 3β alcohol affords 11α-alkenyl 3β-hydroxylandrost-5-en-17-one 6 . If 6 has R=2′-tri-n-butylstannyl ethenyl then lead tetraacetate oxidation affords 11α-alkynyl 3β-hydroxyandrost-5-en-17-one, 6 , (R=C≡CH).

Alkynylation and Alkenylation at Carbon-16

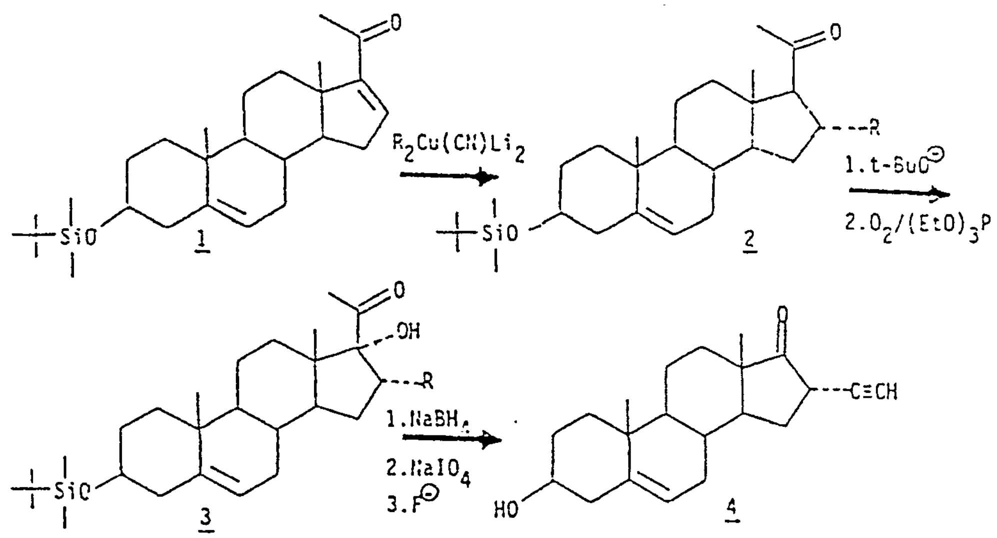


Michael addition of a suitably substituted organo copper reagent such as 2-tri-n-butylstannyl ethenyl 1′-pentynyl lithium cuprate ([$C_3H_7C{\equiv}C$-Cu-CH = CHSn $nBu_3$]Li) to 3β-hydroxypregna-5,16-dien-20-one 3-t-butyl dimethylsilyl ether 1 yields a 16α-tri-n-butylstannyl ethylene (2 , R = CH = CHSn $nBu_3$). Lead tetraacetate oxidation proceeds with the loss of tin and yields the corresponding acetylide. Treatment of 2 with t-butoxide followed by oxygen to generate a C-16α-hydroperoxide which is reduced by triethylphosphite to 16α-ethynyl-3β,17α-dihydroxy-pregna-5-en-20-one 3-t-butyldimethylsilyl ether 3 . Reduction of the C-20 ketone to an alcohol followed by cleavage of the diol with sodium periodate and deprotection of the 3β-hydroxyl group with fluoride, yields 16α-ethynyl-3β-hydroxyandrost-5-en-17-one, 4 . Careful reduction of the acetylene in 4 should afford the 16α-vinyl substituted steroids. Higher homologues of these substituents may be synthesized via similar routes.

The following procedures illustrate hydroxylation at Carbon-1, 2, 4, 7, 11 or 16.

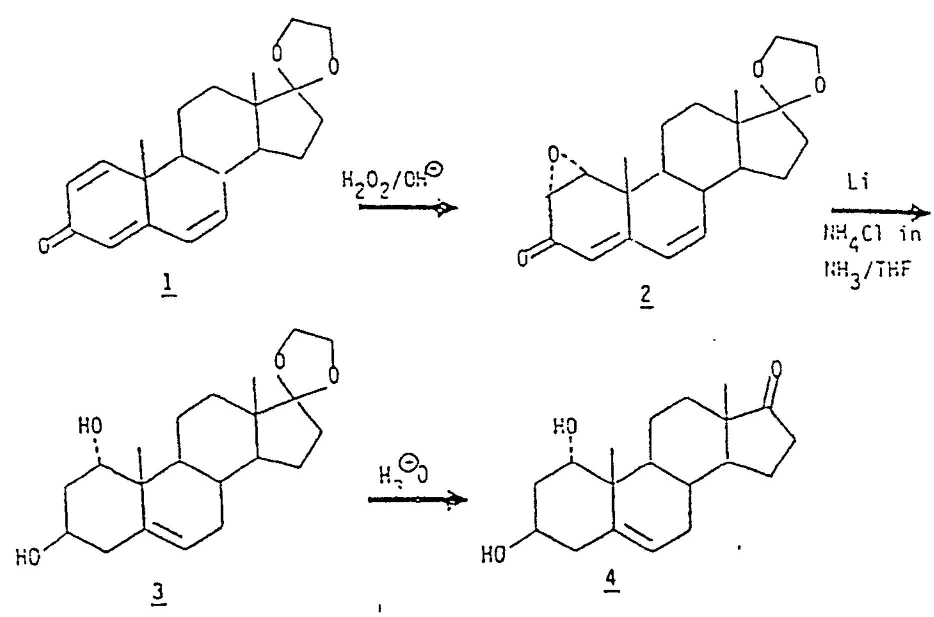


Alkaline hydrogen peroxide epoxidation of androsta-1,4,6-triene-3,17-dione 17-ketal 1 with basic hydrogen peroxide yields the 1α,2α-epoxide 2. Treatment of 1α,2α-epoxyandrosta-4,5-dien-3,17-dione 17-ketal 2 with a large excess each of lithium metal and ammonium chloride in ammonia-tetrahydrofuran (1:1) at reflux leads to 1α,3β-dihydroxyandrost-5-en-17-one 17-ketal 3 . Hydrolysis of the ketal affords 1α,3β-dihydroxyandrost-5-en-17-one, 4 . Also, fermentation of DHEA with penicillium aspergillus affords 4 , i.e.

penicillium aspergillus may be able to 1α-hydroxylate other substrates.

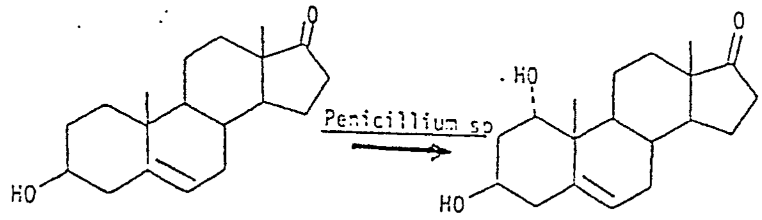


Dodson, R.M., Goldkamp, A.M., and Muir, R.D., JACS , 1957, 79 , 3921.
Dodson, R.M., Goldkamp, A.M., and Muir, R.D., JACS , 1960, 82 , 4026.

Penicillium hydroxylates DHEA at C-1 in the α-position. Therefore, other substrates that look like DHEA should by hydroxylated at C-1 by this enzyme.

C-2 Hydroxylation

2α,3β-dihydroxyandrost-5-en-17-one

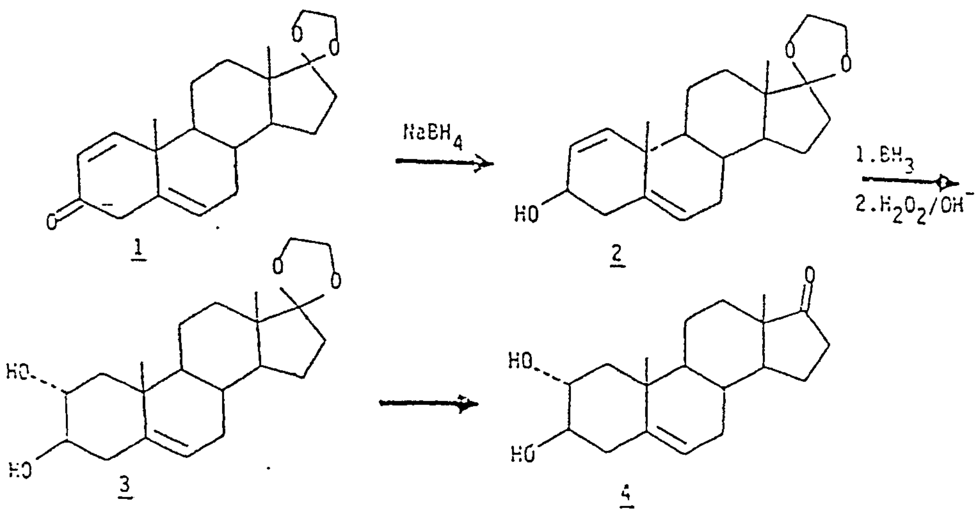


1 2 3 4

Reduction of androsta-1,5-dien-3,17-dione-17-ketal 1 with sodium borohydride yields 3β-hydroxyandrosta-1,5-diene-17-one 17-ketal 2 . Hydroxylation of the C-1 double bond by hydroboration followed by oxidation with alkaline hydrogen peroxide affords 2α,3β-dihydroxyandrost-5-en-17-one 17-ketal 3 . Deprotection of the C-17 ketone with aqueous acid yields 2α,3β-dihydroxyandrost-5-en-17-one, 4 .

Carbon-4 Hydroxylation

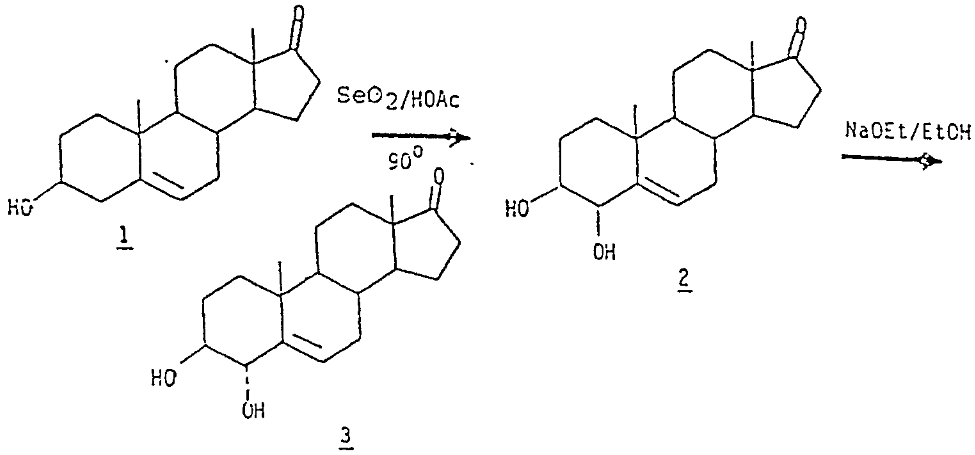


1 2 3

Selenium dioxide oxidation of 3β-hydroxyandrost-5-en-17-one yields 3β,4β-dihydroxyandrost-5-en-17-one 2 . The axial C-4 alcohol may be epimerized to the equatorial position by reaction with sodium ethoxide in ethanol to yield 3β,4α-dihydroxyandrost-5-en-17-one, 3 .

Carbon-7 Hydroxylation

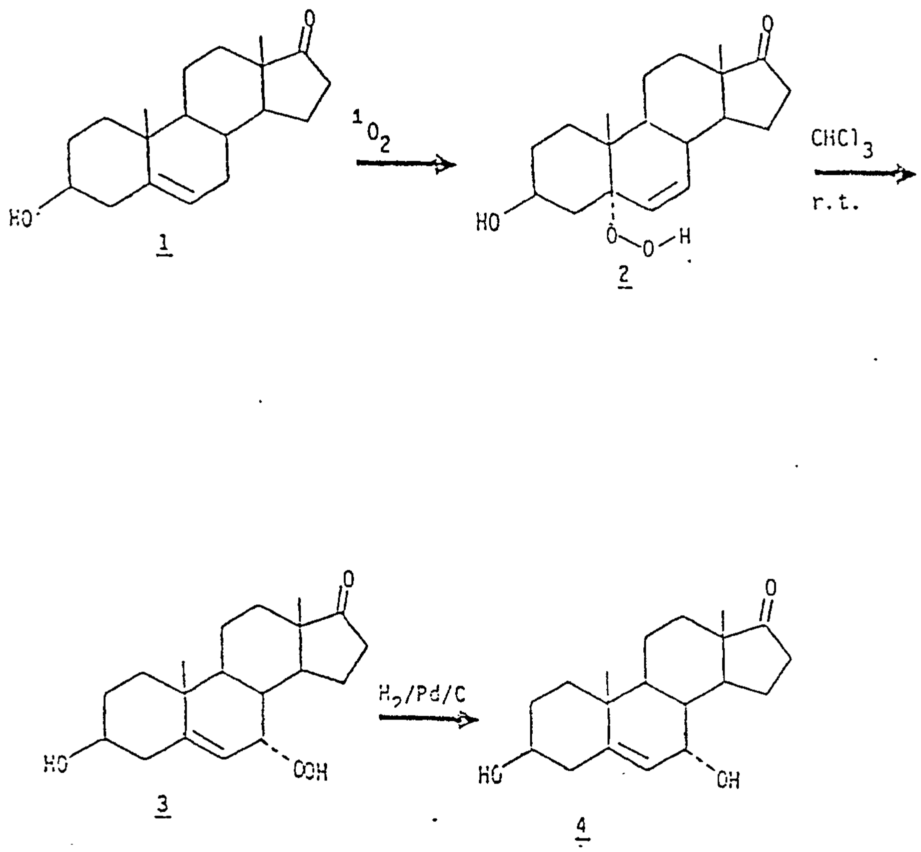


1 2 3 4

3β-Hydroxandrost-5-en-17-one (DHEA) 1 reacts with singlet oxygen to yield 5α-hydroperoxy-3β-hydroxyandrost-6-en-17-one 2 . This hydroperoxide undergoes a rearrangement when in chloroform solution to yield 7α-hydroperoxy-3β-hydroxyandrost-5-en-17-one, 3 . Catalytic reduction of the hydroperoxide yields

3β,7α-dihydroxyandrost-5-en-17-one, 4 .

Carbon-11 Hydroxylation

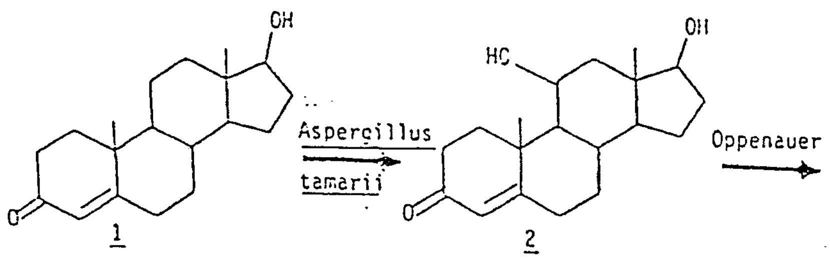


1 2

D.R. Brannon, J. Martin, A.C. Ochlschlager, N.N. Durham, and L.H. Zalkow, J. Org. Chem. 1965. 30, 760.

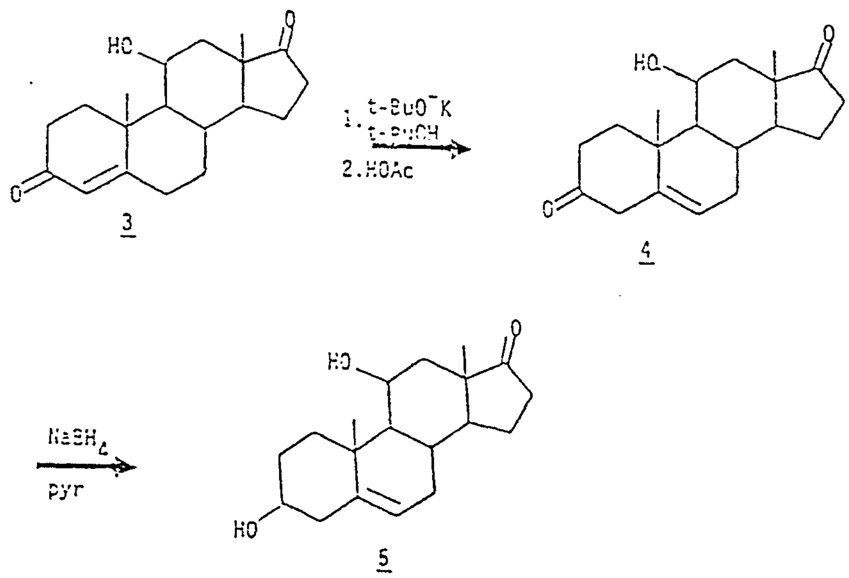


3 4 5

Hydroxylation of testosterone 1 at Carbon-11 using Aspergillus tamarii affords 11β,17β-dihydroxyandrost-4-en-3-one 2 . Oppenauer oxidation of 2 oxidizes the 17β-alcohol in the presence of the hindered 11β-hydroxyl group to yield 11β-hydroxyandrost-4-en-3,17-dione, 3 . Migration of the double bond out of conjunction by treatment with potassium t-butoxide followed by protonation with acetic acid yields 11β-hydroxyandrost-5-en-3,17-dione 4 . Selective reduction of 4 yields 3β,11β-dihydroxyandrost-5-en-17-one, 5 .

Hydroxylation at Carbon-16

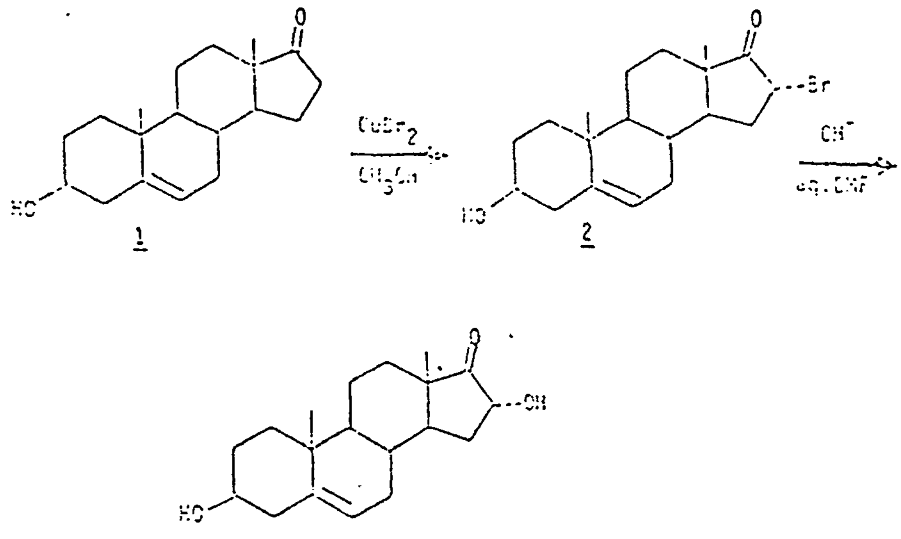


1 2 3

Bromination of DHEA (1 ) with cupric bromide yields 16α-bromo-DHEA, 2 . Treatment of the bromo ketone 2 with sodium hydroxide in aqueous dimethylformamide gave 3β,16α-dihydroxyandros -5-en-17-one, 3 . See M. Numazawa, M. Nagaoka, Y. Osawa, J. Org. Chem. 1982, 47,4024. Similarly 3-β-methyl-16α hydroxy androst-5-en-17-one may be prepared by hydroxylation of 3-β methyl androst-5-en-17-one using this procedure to introduce the 16α-hydroxy group, as illustrated by the following procedure:

To prepare 3β-methyl-16α-bromo-androst-5-ene-17-one (Ref: E. R. Glazier, J. Org. Chem. (1962) 27 2937; M. Numazawa & Y. Osawa, J. Org. Chem. Steriods (1978) 32 , 519; M. Numazawa, M. Nagaoka & Y. Osawa, J. Org. Chem. (1982), 47 4024).

3β-methyl androst-5-ene-17-one (4.0g, 14 mmol) and $CuBr_2$ (9.4g, 42mmol) were dissolved in methanol (250ml) and refluxed for 24h. The hot solution was filtered to remove the white precipitate and the filtrate cooled to yield 3.1g (61%) 3βmethyl-16α-bromoandrost-5-ene-17-one. An analytical sample was prepared by passing an ether solution of the steroid through a small plug of neutral alumina. Evaporation and recrystallization from methanol gave white needles, M.P. 193-195° C. Anal. calc. for $C_{20}H_{29}OBr$, C-65.7, H-8.0. Found C-65.54, H-8.11 NMR ($CDCl_3$) d 5.30 (brd, 1H, H-6), 4.52 (t, 1H, 16β-H) .98 (S, 3H, C-19Mc), .90(S, 3H, C-18Me) IR(KBr) 2910, 1735, 1445, 1365, 1020.

3β-methyl-16α-bromo androst-5-ene-17-one (1g, 2.74 mmol) was dissolved in dimethylformamide (90ml). Sodium hydroxide (165 mg, 4.1 mmol) in water (10ml) was added and the solution stirred for 2 h. at room temperature. The solution was poured into 1% HCl (200 ml) and extracted with ethyl acetate (2 X 50 ml). The organic layer was washed with 5% sodium bicarbonate, water then dried over magnesium sulfate and filtered. Evaporation gave a pale yellow solid which was chromatographed on flash silica gel and eluted with ether/hexane (10/90). Recrystallization from ether gave 3β-methyl-16α-hydroxy androst-5-ene-17-one (0.4g, 50%). M.P. 166-168° C. Anal. calc. for $C_{20}H_{30}O_2$, C-79.42, H-9.99.Found C-79.24, H-10.04 NMR (d-6-DMSO) d 5.30 (brd, 1H, H-6), 4.38(t, 1H, 16β-H) [IR(KBr) 3440, 2900, 1735, 1445, 1365, 1010.] 2.0-1.85-(complex).

The following procedures are representative of procedures for halogenation at Carbon-1, 2, 3, 4, 6, 7, 11 or 16.

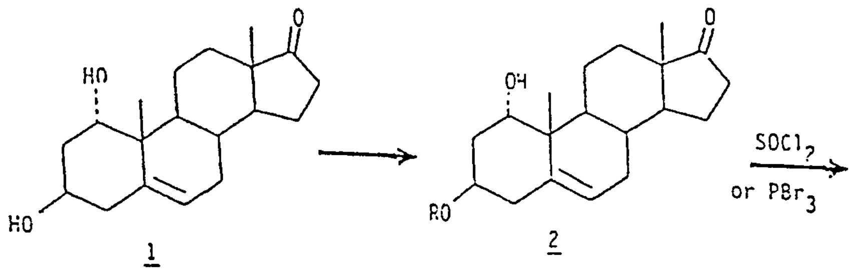


1 2

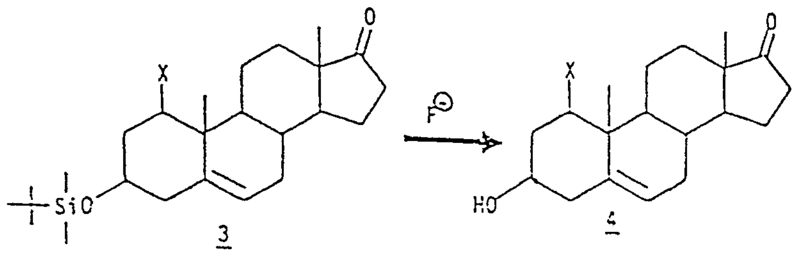


3 4

X = Cl,Br,I

Selective protection of the Carbon-3 hydroxyl in the presence of the 1α-hydroxyl group should yield 2 . For example, 1α,3β-dihydroxyandrost-5-en-17-one 1 reacts with t-butyl-dimethyl silyl chloride in the presence of imidazole using dimethylformamide as a solvent to yield 1α,3β-dihydroxyandrost-5-en-17-one 3t-butyldimethylsilyl ether, 2 . Reaction of 2 with thionyl chloride, or phosphorous tribromide or catechol phosphochloridate followed by iodine yields the corresponding 1β-chloro, bromo or iodo derivatives 3 . Reaction of 3 (R = Cl, Br, I) with tetrabutyl ammonium fluoride yields 1β,-halo-3β-hydroxy androst 5-en-17-one, 4 (R = Cl, Br or I). The fluoride (4 , R = F) may be synthesized via a similar route using an ester as the protecting group at C-3 and reacting the 1α-hydroxyl group with diethyl (2-chloro-1,1,2-trifluoroethyl)amine. Hydrolysis should yield 1,β-fluoro-3β-hydroxyandrost-5-en-17-one, 4 , R = F.

Halogenation at Carbon-2

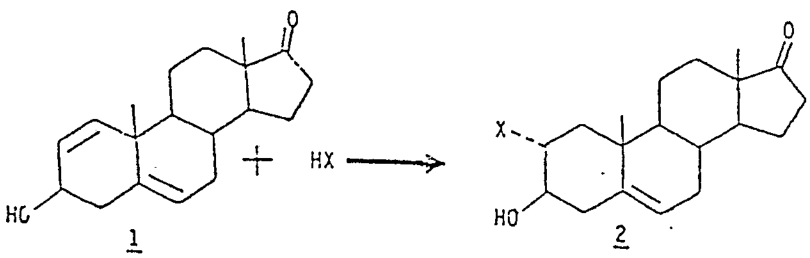

1 2

Addition of HX across the C-1 double bond in 3β-hydroxyandrosta-1,5-diene-17-one, 1 , yields a mixture of the C-1 and C-2 halogenated steroids. Separation affords 2-halo-3β-hydroxyandrost-5-en-17-one (2 , R = F, Cl, Br, I).

Halogenation at Carbon-3

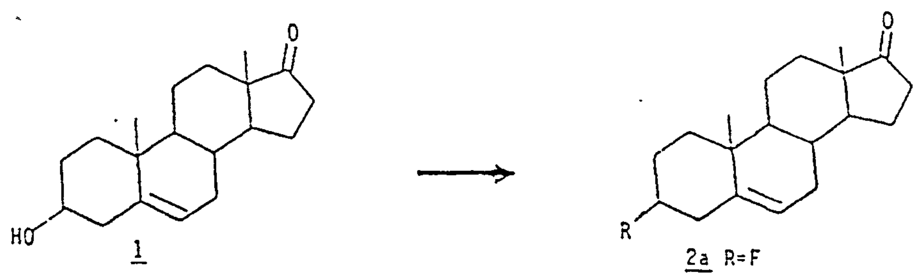

1

2a R=F
b R=Cl
c R=Br
d R=I

Reaction of 3β-hydroxyandrost-5-en-17-one 1 with diethyl (2-chloro-1,1,2-trifluoroethyl) amine yields 3β-fluoroandrost-5-en-17-one 1 . Reaction of 1 with thionyl chloride yields 3β-chloroandrost-5-en-17-one, 2b . Reaction of 1 with phosphorus tribromide yields 3β-bromoandrost-5-en-17-one, 2c . Reaction of 1 with catechol phosphochloridate followed by iodine yields 3β-iodoandrost-5-en-17-one 2d .

Halogenation at Carbon-4

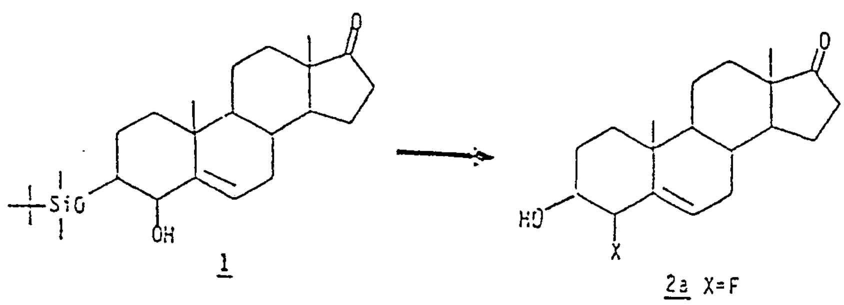

1

2a X=F
b X=Cl
c X=Br
d X=I

With the 3β-hydroxyl group protected as its t-butyl-dimethylsilyl ether the C-4 hydroxyl may be chlorinated using thionyl chloride. Treatment with fluoride ion cleaves the silyl ether to yield 4-chloro-3β-hydroxyandrost-5-en-17-one, 2b . Reaction of 3,4-dihydroxyandrost-5-en-17-one 3-t-butyldimethylsilyl ether 1 with catechol phosphochloridate, followed by displacement with bromide ion and cleavage of the silyl ether with fluoride ion yields 4-bromo-3β-hydroxyandrost-5-en-17-one, 2c . Reaction of 1 with catechol phosphochloridate, followed by iodine and cleavage of the silyl ether with fluoride yields 4-iodo-3β-hydroxyandrost-5-en-17-one, 2d . Fluorination of 3,4-dihydroxyandrost-5-en-17-one 3-acetate with diethyl (2-chloro-1,1,2-trifluoroethyl) amine followed by hydrolysis of the ester yields 4-fluoro-3β-hydroxyandrost-5-en-17-one, 2a .

Halogenation at Carbon-6

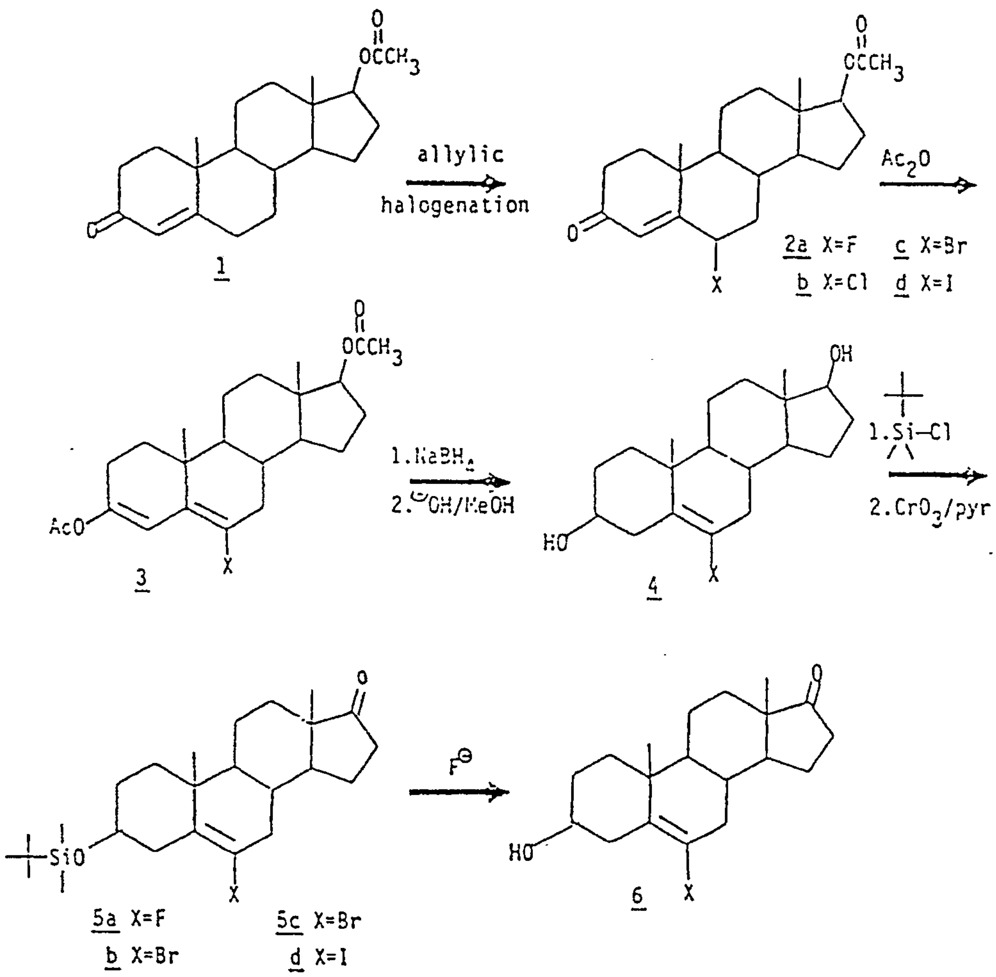


1

2a X=F b X=Cl c X=Br d X=I

3

4

5a X=F b X=Br 5c X=Br d X=I

6

Allylic bromination of 17β-hydroxyandrost-4-en-3-one 17-acetate 1 using N-bromosuccinimide together with a radical initiator such as light or benzoyl peroxides or aliphatic azo compounds [RR′C(CN)-N = N-C-(CN)RR′] e.g. azobisisobutyronitrile yields 6β-bromo-17β-hydroxyandrost-4-en-3-one 17-acetate, 2 . Allylic chlorination of 1 using sulfuryl chloride together with a radical initiator such as light or benzoyl peroxide or aliphatic azo compounds yields 6β-chloro-17β-hydroxyandrost-4-en-3-one 17-acetate, 2c . Allylic iodination of 1 using mercuric iodide and light yields 6β-iodo-17β-hydroxyandrost-4-en-3-one-17-acetate, 2d . Acetylation of 2 with acetic anhydride and p-toluene sulfonic acid in toluene yields 6-halo-3,17β-dihydroxyandrosta-3,5-diene 3,17-diacetate 3 . Sodium borohydride reduction of 3 followed by basic hydrolysis of the C-17 acetate yields 6-haloandrost-5-en-3β,17β-diol, 4 . Selective protection of the C-3 hydroxyl group as its t-butyldimethylsilyl ether followed by chromium trioxide oxidation of the C-17-hydroxyl group yields 6-halo-3β-hydroxyandrost-5-en-17-one 3-t-butyldimethylsilyl ether 5 . Treatment of 5 with fluoride ion yields 6-halo-3β-hydroxyandrost-5-en-17-one, 6 . The C-6 fluoro analogue may be synthesized from the C-6 bromo diacetate, 3c , by treatment with silver fluoride. Reaction of 6-fluoro-3,17β-dihydroxyandrosta-3,5-diene-3,17-diacetate, 3a , with sodium borohydride and following the above sequence yields, 6-fluoro-3β-hydroxyandrost-5-en-17-one, 6a .

Halogenation at Carbon-7

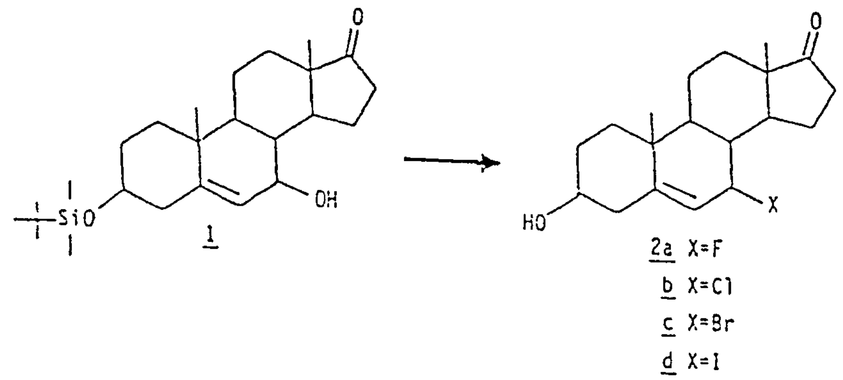

1

2a X=F
b X=Cl
c X=Br
d X=I

Reaction of 3β,7-dihydroxyandrost-5-en-17-one-3-t-butyldimethylsilyl ether 1 with thionyl chloride yields the C-7 chloro-steroid. Deprotection of the 3β-hydroxyl group affords 7-chloro-3β-hydroxyandrost-5-en-17-one, 2b . Reaction of 1 with catechol phosphochloridate followed by displacement with bromide ion and deprotection yields 7-bromo-3β-hydroxyandrost-5-en-17-one, 2c . Similarly reaction of 1 with catechol phosphochloridate followed by displacement with iodine and deprotection yields 7-iodo-3β-hydroxyandrost-5-en-17-one, 2d . Fluorination of 3β,7-dihydroxyandrost-5-en-17-one 3-acetate with diethyl (2-chloro-1,1,2-trifluoro-ethyl)amine followed by hydrolysis of the ester yields 7-fluoro-3β-hydroxyandrost-5-en-17-one, 2a .

Halogenation at Carbon-11

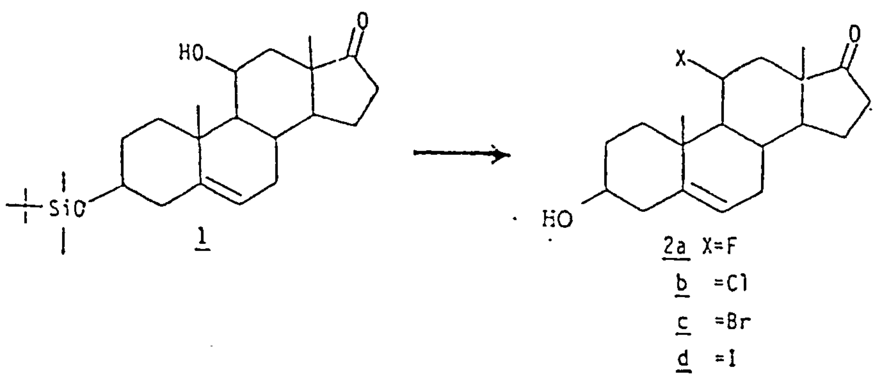

1

2a X=F
b =Cl
c =Br
d =I

Reaction of 3β,11-dihydroxyandrost-5-en-17-one 3-t-butyldimethylsilyl ether 1 with thionyl chloride yields the C-11 chloro steroid. Deprotection of the 3β-hydroxyl group affords 11-chloro-3β-hydroxyandrost-5-en-17-one, 2b . Reaction of 1 with catechol phosphochloridate followed by displacement with bromide ion and deprotection yields 11-bromo-3β-hydroxyandrost-5-en-17-one, 2c . Similarly reaction of 1 with catechol phosphochloridate followed by displacement with iodine and deprotection yields 11-iodo-3β,hydroxyandrost-5-en-17-one, 2d . Fluorination of 3β,11-dihydroxyandrost-5-en-17-one 3-acetate with diethyl (2-chloro-1,1,2-trifluoroethyl)amine followed by hydrolysis of the esteryields 11-fluoro-3β-hydroxyandrost-5-en-17-one, 2a .

Halogenation at Carbon-16

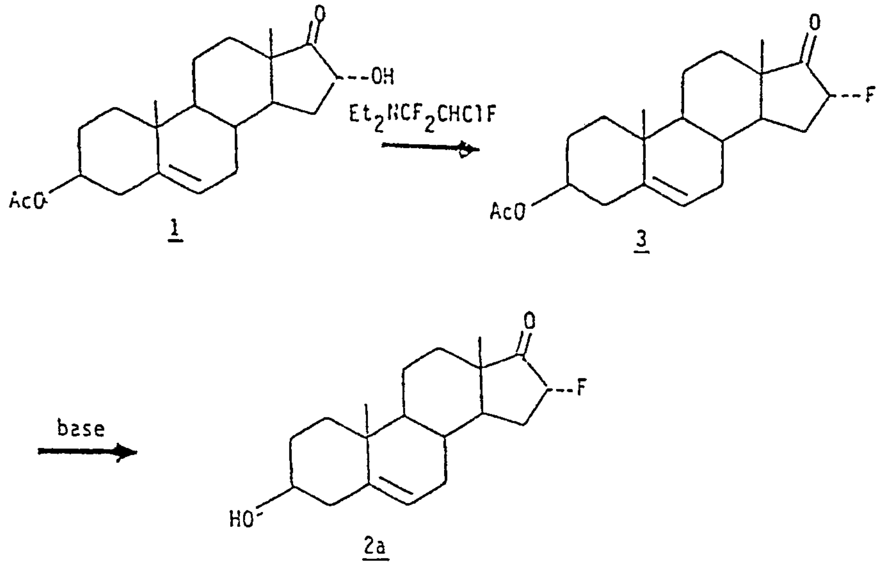


Reaction of 3β,16α-dihydroxyandrost-5-en-17-one 3β-acetate 1 with diethyl (2-chloro-1,1,2-trifluoroethyl)amine affords 16α-fluoro-3β-hydroxyandrost-5-en-17-one 3-acetate 3 . Hydrolysis of the ester with base yields 16α-fluoro-3β-hydroxyandrost-5-en-17-one, 2a .

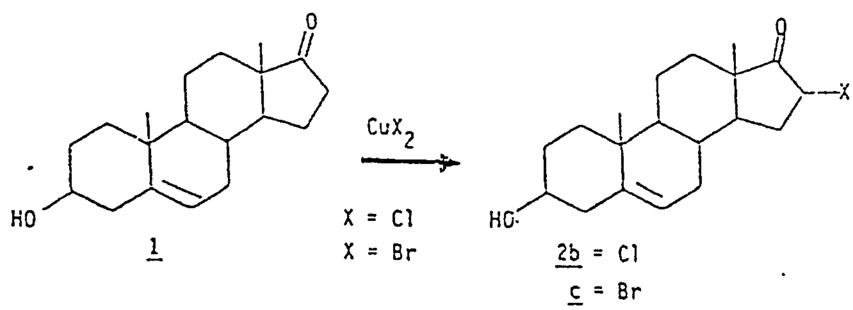


Reaction of 3β-hydroxyandrost-5-en-17-one 1 with cupric bromide yields 16α-bromo-3β-hydroxyandrost-5-en-17-one, 2c [1] . Similarly reaction of 1 with cupric one, 2b .

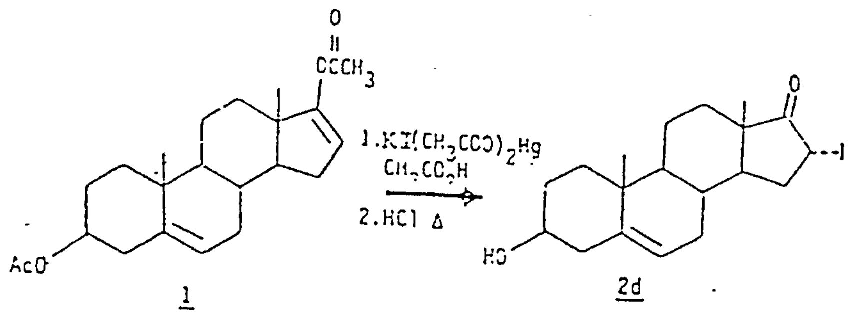


Reaction of 3β,17-dihydroxyandrosta-5,16-diene 17-acetate 1 with mercuric acetate followed by treatment with potassium iodide yielded the C-17 α iodide which hydrolyses with acid to yield 3β-hydroxy-16α-iodoandrost-5-en-17-one, 2d . Reaction of 2d with silverfluoride yields 3β-hydroxy-16α-fluoroandrost-5-en-17-one, 2a .

[1]E. R. Glazier J. Org. Chem. 1962, 27 , 4397

The following procedures are illustrative for the preparation of compounds of the present invention encompassed by the structure:

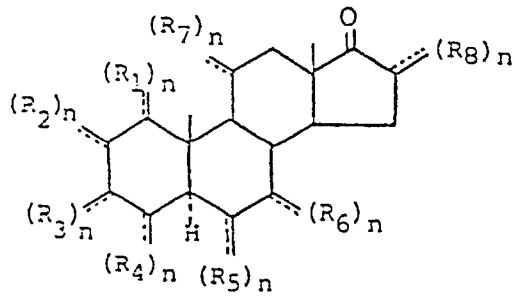

wherein $R_1$, $R_2$, $R_3$, $R_4$, $R_5$, $R_6$, $R_7$, $R_8$ and n are as defined hereinbefore.

Catalytic hydrogenation of 3β-substituted androst-5-enes yields almost exclusively 3β-substituted 5α-androstanes (for references see J. R. Lewis and C. W. Shoppee, J. Chem. Soc. 1955, 1365). Therefore all the syntheses of the substituted androst-5-enes described above can be used for the synthesis of the substituted 5α-androstanes, except those molecules which contain reducible double bonds such as the ethenyl and alkynyl derivatives. For these molecules the following syntheses are described.

Firstly an example of catalytic hydrogenation for the synthesis of 5α-androstanes from androst-5-enes is the synthesis of 3β-methyl-5α-androstan-17-one 2 from 3β-methylandrost-5-en-17-one 1 . 3β-Methylandrost-5-en-17-one 1 (400 mg), prepared as described previously was dissolved in glacial acetic acid (80 ml). Palladium on carbon (10%, 100 mg) was added and the solution maintained under an atmosphere of hydrogen. When hydrogen uptake ceased, the solution was filtered through celite and evaporated to give solid which was recrystallized from methanol to yield 3β-methyl-5α-androstan-17-one, 2 , (320 mg, 80% yield). MP 107-108° C, $^1H$ NMR ($CDCl_3$) δ 0.86 (d, 3H, J-5Hz, methyl at C-3), 0.85 (s, 3H, C-19 Me), 0.79 (s, 3H, C-18 Me).

| | | |
|---|---|---|
| Anal Calc for $CH_{32}O$: | C, 83.26%, | H 11.18% |
| Found: | C, 82.99%, | H 11.35% |

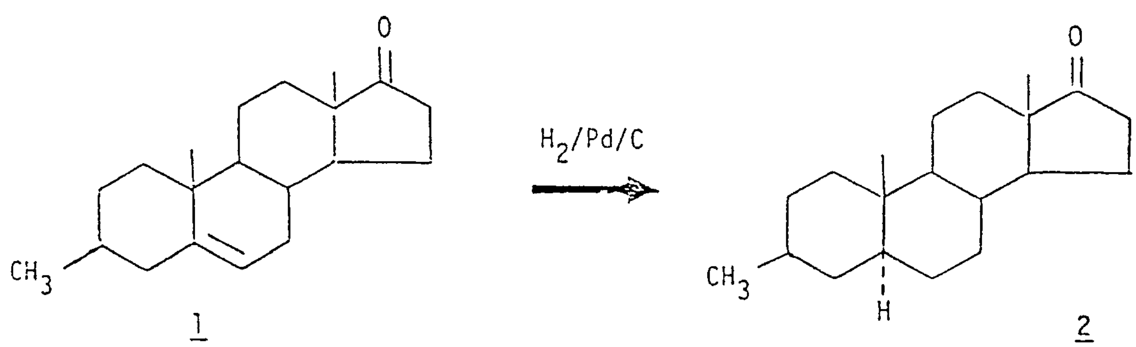


1　　　　　　　　2

The following procedures are illustrative for alkenylation and alkynylation at carbon-1.

Michael addition to 17β-hydroxy-5α-androst-1-en-3-one 17-acetate, 1 , using a dialkenyl lithium cuprate, ($RR^1CuLi$, R = $R^1$ = $CH=CHR^2$) yields the 1α-alkenyl-17β-hydroxy-5α-androstan-3-one 17-acetate 2 . Reduction of the C-3 ketone in 2 yields the 3β-hydroxy steroid 3 . Protection of the 3β-hydroxyl group as a dimethyl-t-butylsilyl ether followed by hydrolysis of the C-17 acetate yields 1α-alkenyl-3β, 17β-dihydroxy-5α-androstan 3-dimethyl-t-butylsilyl ether, 4 . Oxidation of the C-17-hydroxyl group and deprotection of the 3β-hydroxyl group with fluoride ion affords 1α-alkenyl-3β-hydroxy-5α-androstan-17-one, 5 . (R = $CH=CHR'$ where $R'$ = alkyl). Alkynylation, to prepare 5 (R = $C\equiv CR'$ where $R'$ = alkyl) maybe carried out using the

above procedure but with a different organo cuprate reagent. Using 2-tri-n-butylstannyl ethenyl 1′-pentynyl lithium cuprate (R R′CuLi is equivalent to [$C_3H_7C{\equiv}C$-Cu-CH=$CHSnnBu_3$]Li), (E. J. Corey and R. H. Wollenberg, J. Amer. Chem. Soc. 1974, 96 , 5581), tri-n-butylstannylethylene is added to 1 to yield 2 with R = CH=CHSnn-$Bu_3$. Oxidation using lead tetraacetate proceeds with loss of tin and affords the corresponding acetylide 2 , (R= C≡CH). Following through the reaction sequence as above yields 1α-ethenyl-3β-hydroxy-5α-androstan-17-one 5 , R = C≡CH.

Alkenylation and Alkynylation at Carbon-1.

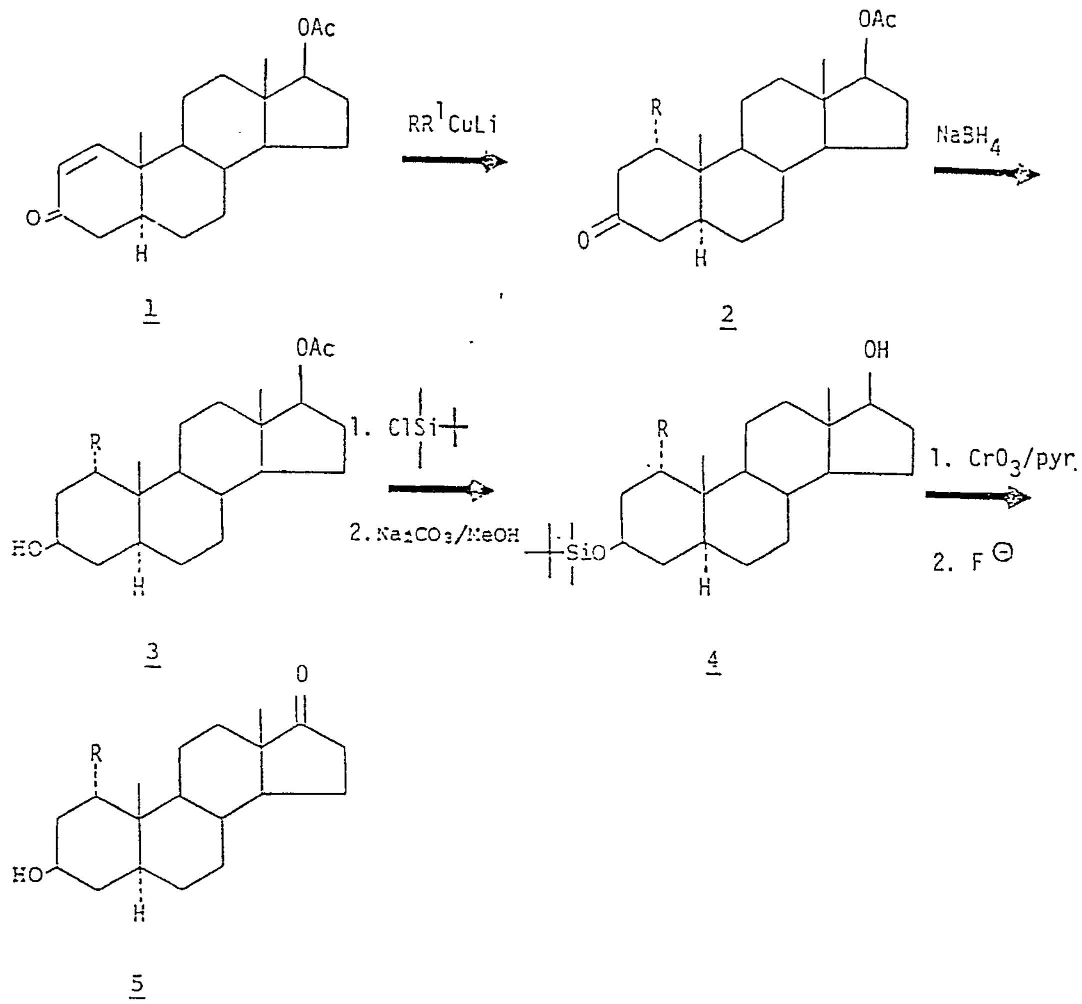


Alkenylation and Alkynylation at Carbon-2.

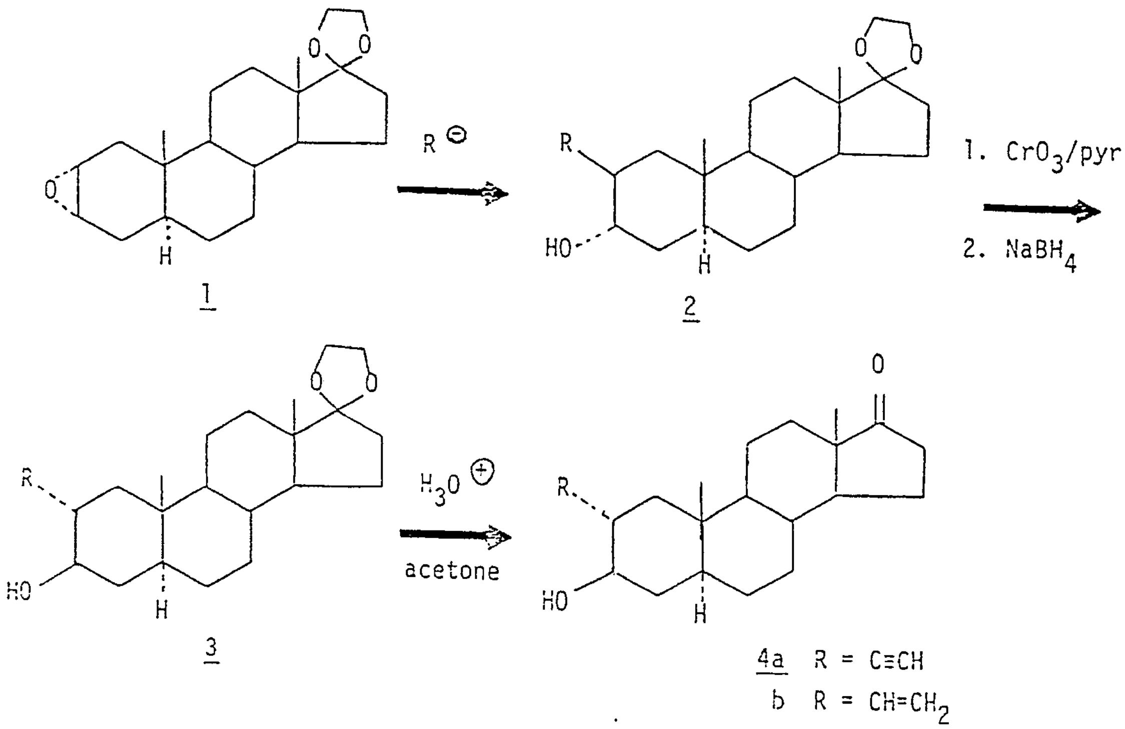


1

2

3

4a R = C≡CH

b R = CH=CH$_2$

Reaction of 2α, 3α-epoxy-5α-androstan-17-one 17-ketal 1 with lithium acetylide ethylene diamine complex yields 2β-ethynyl-5α-androstan-3 -ol-17-one 17-ketal 2 , (R = C≡CH). Epimerization of the C-3 alcohol by oxidation to the C-3 ketone (chromium trioxide/pyridine) and reduction with sodium borohydride affords 2β-ethynyl-5α-androstan-3β-ol-17-one 17-ketal 3 (R = C≡CH). Deprotection of the 17-ketone by treatment with aqueous acid yields 2α-ethynyl-5α-androstan-3β-ol-17-one 4a . The 2α-ethenyl steroid can be synthesized from the ethynyl derivative by careful catalytic reduction with Lindlar catalyst to yield 2α-ethenyl-3β-hydroxy-5α-androstan-17-one, 4b .

Alkenylation and Alkynylation at Carbon-3.

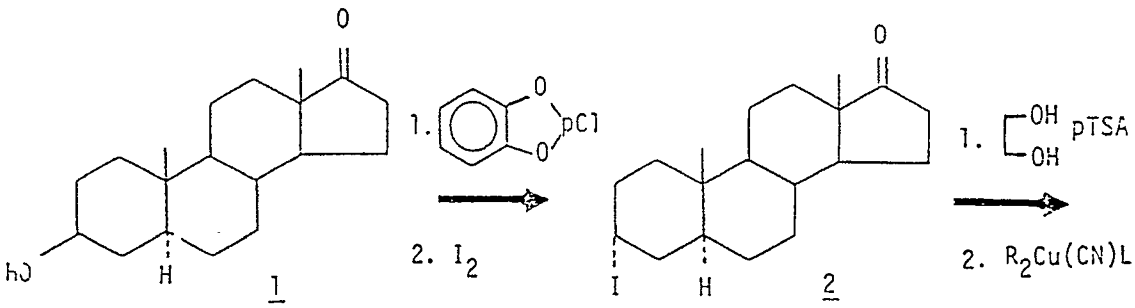


1 2

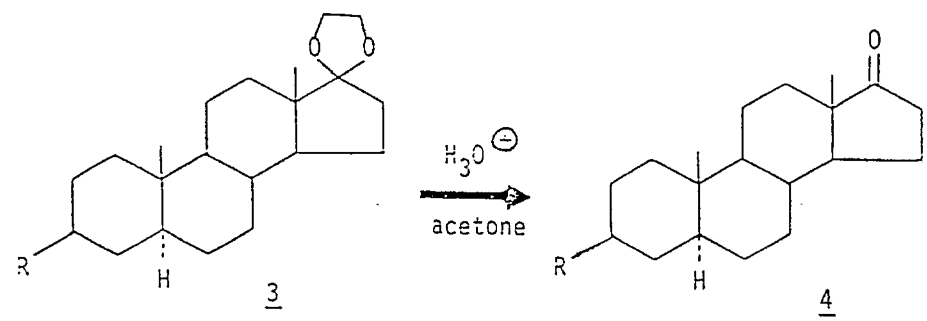


3 4

Reaction of 3β-hydroxy-5α-androstan-17-one 1 with catechol phosphochloridate followed by iodine affords 3α-iodo-5α-androstan-17-one, 2 . Protection of the C-17 ketone as a ketal followed by nucleophilic displacement of the C-3α-iodo group yields the 3β-substituted steroid 3 . For alkenylation at C-3, such as introduction of a vinyl group, i.e. 3 (R = CH=CH$_2$), divinyl cyano dilithio cuprate, [(CH$_2$=CH)$_2$ Cu(CN)]Li$_2$ is used. Hydrolysis of the C-17 ketal yields 3β-ethenyl-5α-androstan-17-one, 4 (R = CH=CH$_2$). For alkynylation 2-tri-n-butylstannyl ethenyl 1′-pentynyl cyano dilithio cuprate, [C$_3$H$_7$C≡C-Cu-CH=CHSn-nBu$_3$-(CN)]Li$_2$, yields the 3β-ethynyl derivative 3 (R = C≡CH). Hydrolysis of the C-17 ketal yields 3β-ethynyl-5α-androstan-17-one, 4 ,(R = C≡CH).

Alkenylation and Alkynylation at Carbon-4.

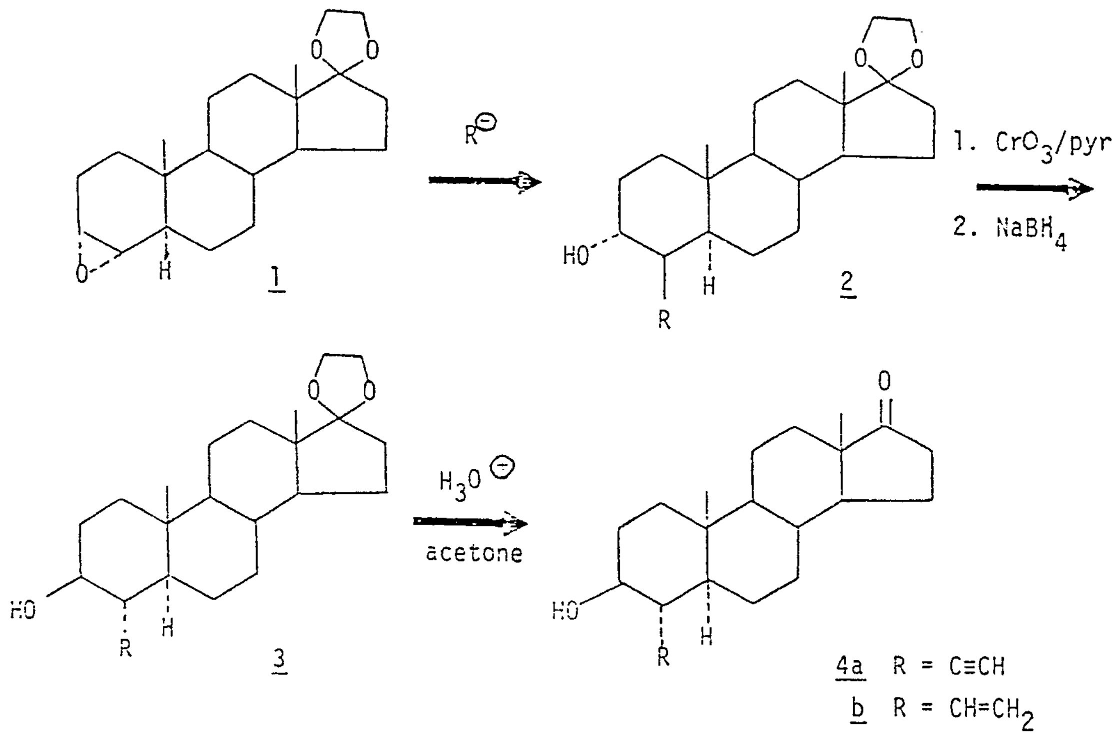


Reaction of 3α,4α-epoxy-5α-androstan-17-one 17-ketal 1 with lithium acetylide diethylamine complex affords 4β-ethynyl-3α-hydroxy-5α-androstan-17-one 17-ketal 2 (R = C≡CH). Epimerization of the C-3 alcohol by oxidation to the C-3 ketone with chromium trioxide/pyridine followed by reduction with sodium borohydride affords 4α-ethynyl-3β-hydroxy-5α-androstan-17-one 17-ketal 3 (R = C≡CH). Careful hydrolysis of the C-17 ketal affords 4α-ethynyl-3β-hydroxy-5α-androstan-17-one, 4a (R = C≡CH). The 4α-ethenyl derivative, 4b , can be synthesized from the ethynyl derivative, 4a , by careful catalytic reduction with Lindlar catalyst or metal ammonia reduction to yield 4α-ethenyl-3β-hydroxy-5α-androstan-17-one, 4b .

Alkenylation and Alkynylation at Carbon-6.

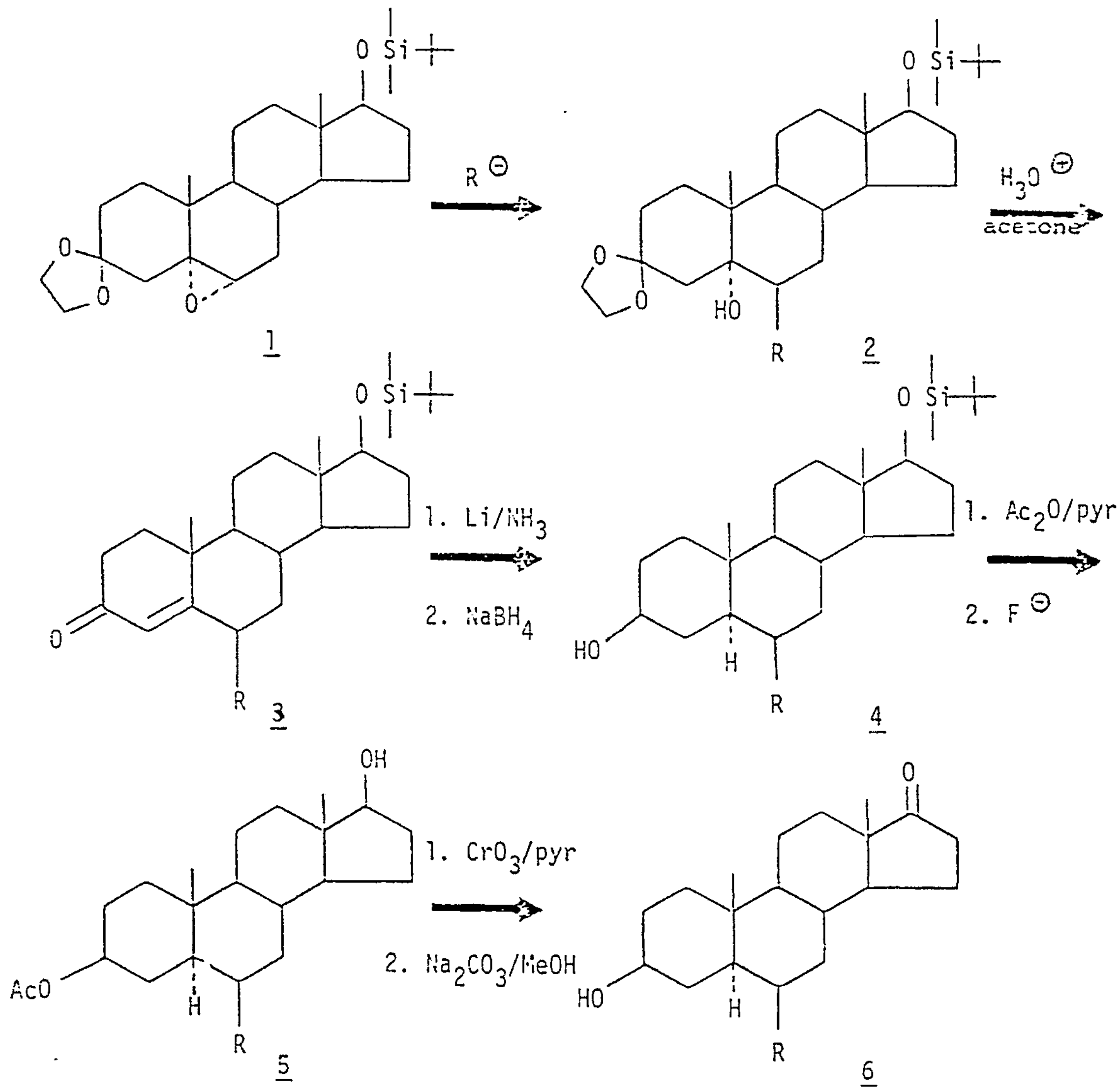


Reaction of 5α,6α-epoxy-3β-dimethyl-t-butylsilyl-oxyandrostan-3-one 3-ketal 1 , with lithium acetylide-ethylenediamine complex yields 6β-ethynyl-5α-hydroxy-3β-dimethyl-t-butylsilyloxyandrostan-3-one 3-ketal, 2 . Hydrolysis of the C-3 ketal and dehydration of the consequent β-hydroxy ketone yields the enone 3 . If the C-17 silyl group is lost under these hydrolysis conditions the C-17 hydroxyl group will be reprotected. Reduction of 6β-ethynyl-17β-dimethyl-t-butylsilyloxyandrost-4-en-3-one, 3 , with excess lithium in ammonia followed by rapid quenching with ammonium chloride affords primarily the 3-keto-4,5α-dihydro compound. (A. Bowers, H.J. Ringold, and E. Denot, J. Amer. Chem. Soc. 1958, 80 , 6115). Sodium borohydride reduction of the C-3 ketone yields 6β-ethynyl-5α-androstan-3β,17β-diol 17-dimethyl-t-butylsilyl ether, 4 . Protection of the C-3 alcohol as an acetate and deprotection at C-17 with fluoride ion yields 6β-ethynyl-5α-androstan-3β,17β-diol 3-acetate, 5 . Oxidation of the C-17 hydroxyl group with chromium trioxide/pyridine followed by deprotection of the C-3-hydroxyl group yields 6β-ethynyl-5α-androstan-3β-ol-17-one, 6 . Higher homologues can be synthesized from 6 by first protecting the ketone and alcohol then using the acetylide anion to react with primary alkyl halides. The C-6 ethenyl derivates (6 R = $CH=CH_2$) can be prepared by reduction of the corresponding C-6 ethynyl derivatives.

Alkenylation and Alkynylation at Carbon-7.

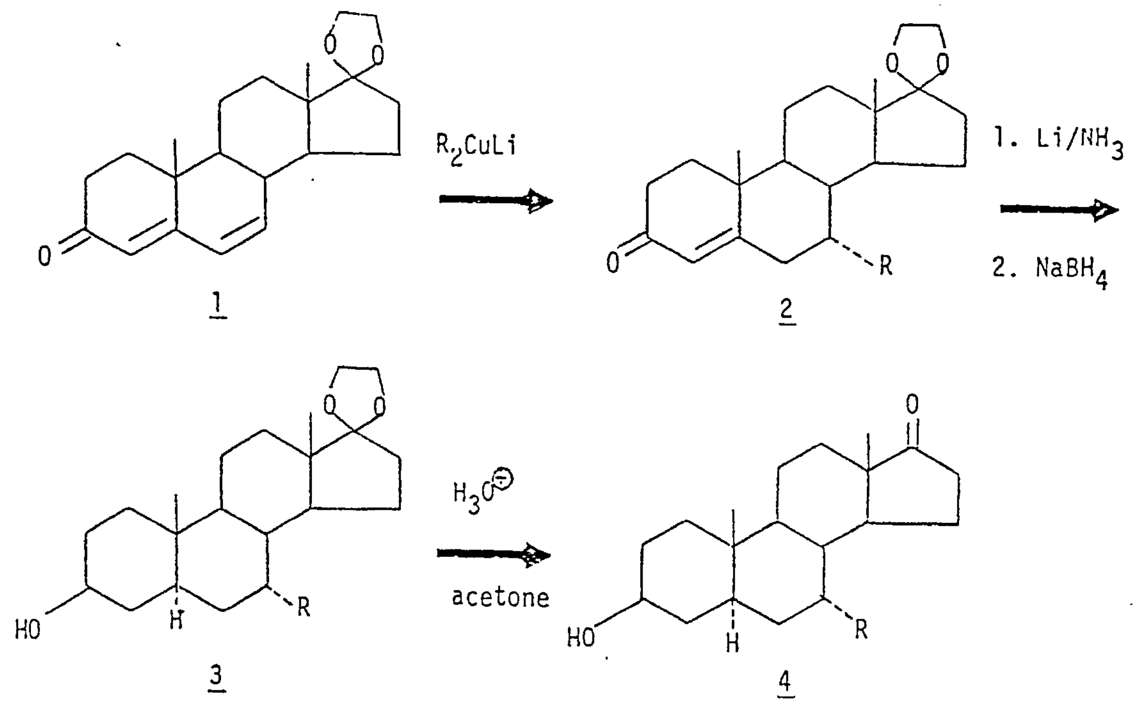


Alkenylation of androsta-4,6-dien-3,17-dione 17-ketal 1 with 2-tri-n-butylstannyl ethenyl 1′-pentynyl lithium cuprate ($[C_3H_7C{\equiv}C\text{-}Cu\text{-}CH{=}CHSn\ nBu_3]Li$) yields the 7α-alkenyl steroid 2 (R = $CH{=}CHSn\ nBu_3$). Oxidation using lead tetraacetate proceeds with the loss of tin affords the corresponding acetylene 2 )R = $C{\equiv}CH$). Reduction of 7α-ethynylandrost-4-en-3,17-dione 17-ketal 2 , (R = $C{\equiv}CH$), with excess lithium in ammonia followed by rapid quenching with ammonium chloride affords primarily the 3-keto-4,5α-dihydro compound. Sodium borohydride reduction of the C-3 ketone yields 7α-ethynyl-5α-androstan-3β-ol-17-one 17-ketal 3 . Careful acid hydrolysis of the C-17 ketal yields 7α-ethynyl-5α-androstan-3β-ol-17-one 4 (R = $C{\equiv}CH$). Higher homologues can be synthesized from 4 , by first protecting the ketone and alcohol, then using the acetylide anion to react with primary alkyl halides. The C-7 ethenyl derivatives 4 (R = $CH{=}CH_2$) can be prepared by reduction of the corresponding C-7 ethynyl derivatives.

Alkenylation and Alkynylation at Carbon-11.

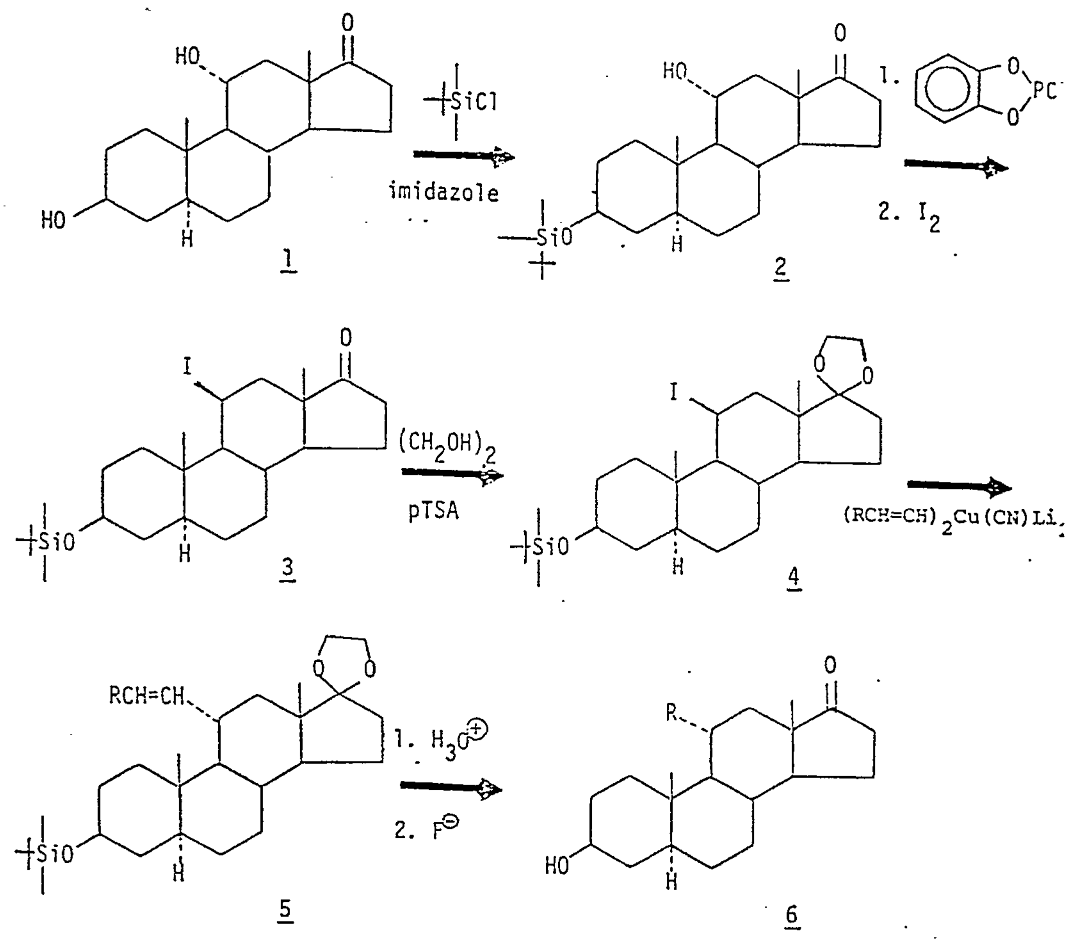


1 2 3 4 5 6

Reaction of the less hindered 3β-hydroxy-5α-androstan-17-one 1 with t-butyldimethylsilyl chloride yields the 3β-t-butyldimethylsilyl ether 2 . Treatment of this first with catechol phosphochloridate followed by displacement with iodine yields 3β-hydroxy-11β-iodo-5α-androstan-17-one 3-dimethyl-t-butylsilyl ether 3 . Protection of the C-17 ketone as the 1,3-dioxolane 4 followed by alkenylation using dialkenyl dilithio cyano cuprate, $(RCH=CH)_2Cu(CN)Li_2$ yields 11α-alkenyl-3β-hydroxy-5α-androstan-17-one-3-t-butyldimethylsilyl ether 5 . Deprotection of the C-17 ketone and 3β alcohol affords 11α-alkenyl 3β-hydroxy-5α-androstan-17-one 6 . If 6 has R=2′-tri-n-butylstannyl ethenyl, then lead tetraacetate oxidation affords 11α-alkynyl-3β-hydroxy-5α-androstan-17-one 6 , (R= C≡CH).

Alkenylation and Alkynylation at Carbon-16.

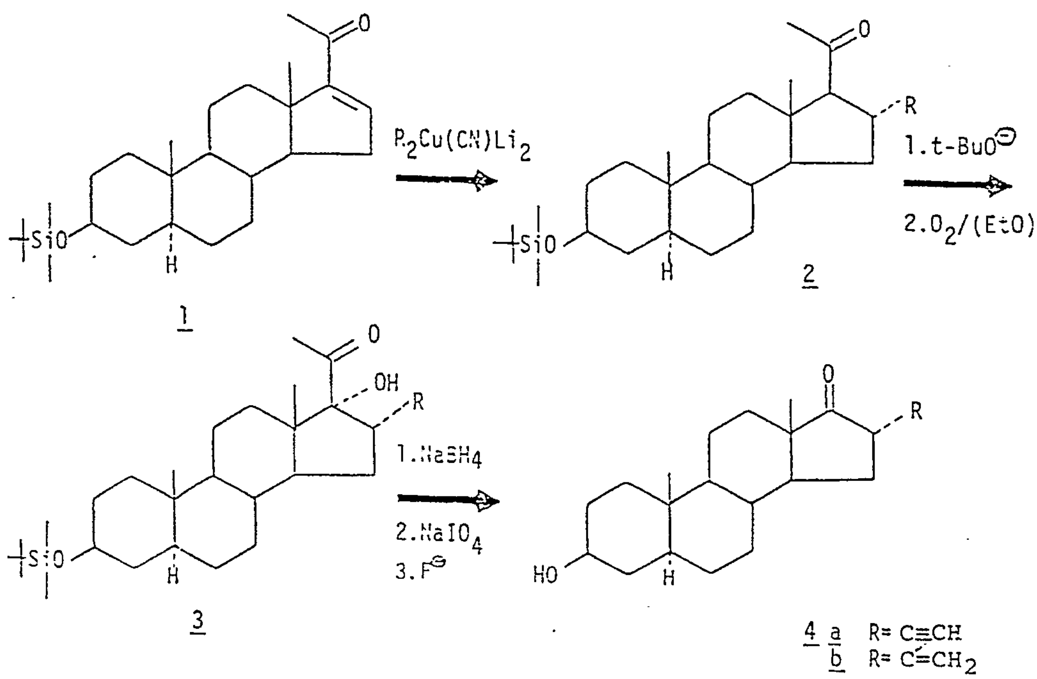


1

2

3

4 a R= C≡CH
b R= C=CH2
c R= C≡CR
d R= CH=CHR1

Michael addition of a suitably substituted organo copper reagent such as 2-tri-n-butylstannyl ethenyl 1′-pentynyl lithium cuprate ([$C_3H_7C{\equiv}C$-Cu=CH=CHSn $nBu_3$]Li) to 3β-hydroxy-5α-pregna-16-en-20-one 3-t-butyl dimethylsilyl ether 1 yields a 16α-tri-n-butylstannyl ethylene (2 , R= CH-CHSn $nBu_3$). Lead tetraacetate oxidation proceeds with the loss of tin and yields the corresponding acetylide. Treatment of 2 with t-butoxide followed by oxygen generates a C-16α-hydroperoxide which is reduced by triethylphosphite to 16α-ethynyl-3β,17α-dihydroxy-5α-pregnan-20-one 3-t-butyldimethylsilyl ether 3 . Reduction of the C-20 ketone to an alcohol followed by cleavage of the diol with sodium periodate and deprotection of the 3β-hydroxyl group with fluoride, yields 16α-ethynyl-3β-hydroxy-5α-androstan-17-one, 4a (R = C≡CH). Careful reduction of the acetylene in 4a should afford the 16α-vinyl substituted steroids, 4b (R = $CH=CH_2$). Higher homologues of these substituents may be synthesized via acetylide chemistry using 4a with its hydroxyl and ketone groups first protected. Reduction of the substituted acetylene will afford both E and Z olefinic substituents at C-16.

In order to determine the pharmacological activity of the novel and other steroids of the present invention, the following experiments are carried out.

## Inhibition of G6PDH

The compounds are screened as inhibitors of purified bovine adrenal G6PDH activity as one predictor of cancer preventive action. The results are shown in Table I:

Table I

| Compound | No. | Conc. | Per Cent Inhibition |
|---|---|---|---|
| 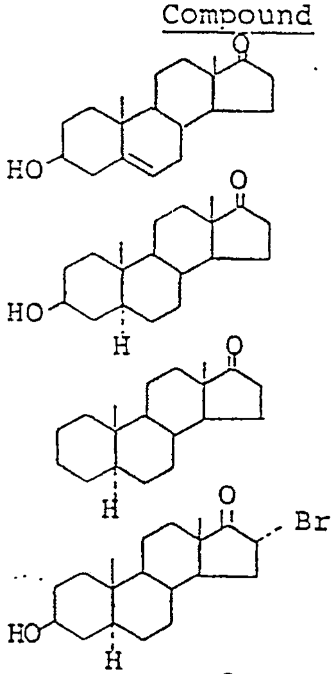 | 1 | 10 µm | 53 |
| | | 1 µm | 36 |
| | | 0.1 µm | 12 |
| | 2 | 10 µm | 82 |
| | | 1 µm | 64 |
| | | 0.1 µm | 36 |
| | 3 | 10 µm | 80 |
| | | 1 µm | 69 |
| | | 0.1 µm | 10 |
| | 4 | 10 µm | 90 |
| 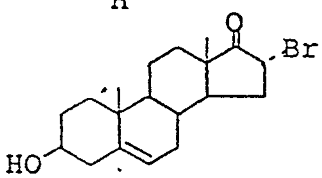 | 5 | 10 µm | 74 |

| Compound | No. | Conc. | Per Cent Inhibition |
|---|---|---|---|
| 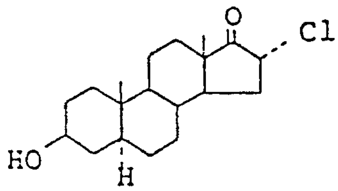 | 6 | 10 µm | 72 |
| 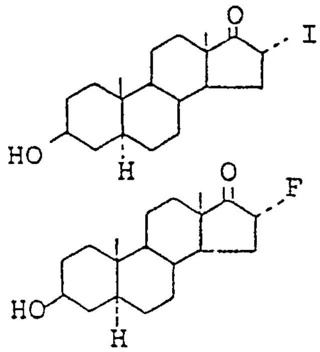 | 7 | 10 µm | 51 |
| | 8 | 10 µm | 66 |
| 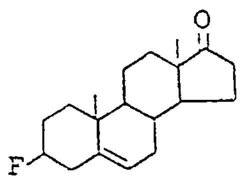 | 9 | 10 µm | 22 |
| 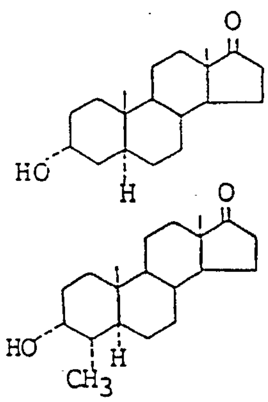 | 10 | 10 µm | 35 |
| | 11 | 10 µm | 35 |

| Compound | No. | Conc. | Per Cent Inhibition |
|---|---|---|---|
| 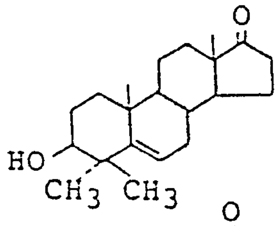 | 12 | 10 μm | 48 |
| 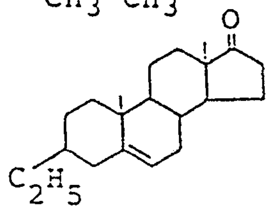 | 13 | 10 μm | 73 |
| | | 1 μm | 55 |
| | | 0.1 μm | 58 |
| 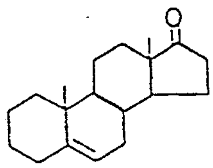 | 14 | 10 μm | 60 |
| | | 1 μm | 15 |
| | | 0.1 μm | 0 |
| 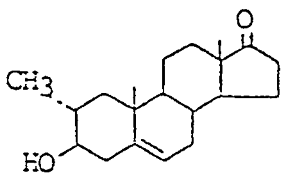 | 15 | 10 μm | 46 |
| | | 1 μm | 40 |
| | | 0.1 μm | 26 |
| 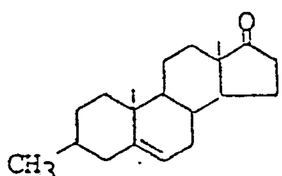 | 16 | 10 μm | 53 |
| | | 1 μm | 40 |
| | | 0.1 μm | 25 |
| 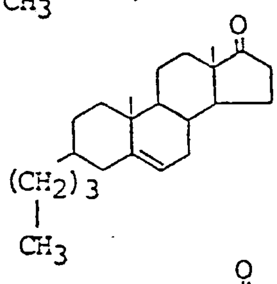 | 17 | 10 μm | 60 |
| | | 1 μm | 45 |
| | | 0.1 μm | 49 |
| 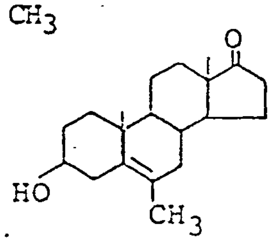 | 18 | 10 μm | 45 |
| | | 1 μm | 18 |
| | | 0.1 μm | 10 |

Inhibition of TPA (Tumor Promoter) Stimulation of Mouse Epidermal DNA

Synthesis rate by orally administered steroids.

The inhibition of tumor promoter stimulation of mouse epidermal DNA synthesis rate by steroids may also contribute to cancer preventive activity. The following assay is used:

ICR male mice (7-9 weeks old) were shaved on the back 1-2 days before use. Only mice showing no hair regrowth were used. Animals were orally intubated with a particular steroid suspended by homogenization in sesame oil or with sesame oil alone (controls). One hour later TPA (10 µg in 0.2 ml of acetone) or acetone vehicle was applied topically to the shaved skin. Twenty hours later, mice were injected i.p. with 60 µCi of $^3$H-thymidine 20 minutes before sacrifice. The animals were killed by cervical dislocation and the residual hair was removed with a depilatory agent. Epidermal scrapings were prepared according to the procedures of Hennings et al. (Cancer Res. 28 , 53, 1968), homogenized in distilled water at 4° C, and the macromolecules precipitated with 0.4 N trichloroacetic acid (TCA). Following 6 washes with absolute ethanol at room temperature, the nucleic acids were hydrolyzed with 0.5 N TCA at 70° C for 5 minutes. The hydrolysate (0.2 ml aliquots) were counted in an Intertechnique scintillation counter and assayed for DNA by the diphenylamine reaction.

The data are expressed as counts per minute (cpm) of tritium per µg of DNA.

| | cpm/µg DNA |
|---|---|
| Control (no TPA and no steroid) | 37 ± 6.1 (number (n) of animals = 3) |
| TPA | 101 ± 20 |
| TPA + DHEA (compound 1)* (400 mg/kg) | 42 ± 7 |
| TPA + DHEA (200 mg/kg) | 88 ± 9.3 |
| TPA + DHEA (100 mg/kg) | 87 ± 8.2 |
| TPA + Compound 2 * (200 mg/kg) | 100 ± 6.0 |
| TPA + Compound 2* (100 mg/kg) | 97 ± 15 |
| TPA + Compound 3 * (200 mg/kg) | 64 ± 10 |
| TPA + Compound 3 * (100 mg/kg) | 113 ± 21 |

* of Table 1

Conclusion:

DHEA is active at blocking the TPA stimulation in DNA synthesis rate at 400 mg/kg but not at 200 mg/kg or 100 mg/kg. Compound 2 is not active at 200 mg/kg or 100 mg/kg. Other tests in which compound 2 or compound 3 and DHEA were given by i.p. injection in a dose-response experiment indicated that 2 is about as active as DHEA and compound 3 was more active in blocking the TPA stimulation in DNA synthesis rate.

Compound 3 appears somewhat more active than DHEA at the dose of 200 mg/kg administered orally.

Compound 2 or 3 in comparison with DHEA by Intraperitoneal Injection.

Steroids were suspended in sterile 95% saline -5% Emulphor and injected intraperitoneally. Otherwise conditions were the same as when steroids were orally administered.

| Compound 2 vs. DHEA | cpm/µg DNA |
|---|---|
| Control (no steroid or TPA) | 63 ± 3.9 (n = 2) |
| TPA | 170 ± 2.2 |
| DHEA (10 mg/kg i.p.) + TPA | 66 ± 2.1 |
| DHEA ( 2 mg/kg i.p.) + TPA | 105 ± 12 |
| DHEA (0.4 mg/kg i.p.) + TPA | 157 ± 4.2 |
| Cpd 2 (10 mg/kg i.p.) + TPA | 58 ± 0.9 |
| Cpd 2 ( 2 mg/kg i.p.) + TPA | 94 ± 1.8 |
| Cpd 2 (0.4 mg/kg i.p.) + TPA | 148 ± 3.0 |

| Compound 3 vs. DHEA | cpm/µg DNA |
|---|---|
| Control (no steroid or TPA) | 46 ± 5.3 |
| TPA | 114 ± 37 |
| Cpd 3 (10 mg/kg i.p.) + TPA | 8.9 ± 3.0 |
| Cpd 3 ( 2 mg/kg i.p.) + TPA | 27 ± 8.8 |
| Cpd 3 (0.4 mg/kg i.p.) + TPA | 32 ± 2.4 |

Compound 16* vs. DHEA

A similar oral dose-response experiment with compound 16 and DHEA was performed.

| | cpm/µg DNA |
|---|---|
| Control (no TPA, no steroid) | 55 ± 3.7 (n = 2) |
| TPA | 162 ± 2.1 |
| TPA + DHEA (400 mg/kg) | 50 ± 2.8 |
| TPA + DHEA (200 mg/kg) | 155 ± 1.6 |
| TPA + DHEA (100 mg/kg) | 169 ± 11 |
| TPA + Compound 16* (400 mg/kg) | 39 ± 1.1 |
| TPA + Compound 16* (200 mg/kg) | 44 ± 2.5 |
| TPA + Compound 16* (100 mg/kg) | 100 ± 19 |

*of Table I

Conclusion:

Compound 16 is about 3X as active as DHEA in this test.

Anti-Obesity Test

Male A/J mice (5 weeks old) were obtained from the Jackson Laboratory and were housed in polycarbonate cages (5 mice/cage) in animal quarters maintained at 24 ± 1 °C with 12 hours of light and 12 hours of darkness each day. One week after arrival, the mice were placed on a chow diet containing varying concentrations of DHEA or other steroid. Animals were weighed weekly; food consumption was determined weekly by subtracting the amount of food remaining in the cage from the amount added.

| Compound 3 vs. DHEA | | | | |
|---|---|---|---|---|
| Week | Control (no steroid) | DHEA 0.7% | Cpd 3 0.35% | Cpd 3 0.7% |
| | | mean weekly weight in grams (n=5) | | |
| 0 | 21.6 ± 2.6 | 21.1 ± 2.8 | 21.8 ± 2.2 | 21.6 ± 2.6 |
| 1 | 22.6 ± 2.1 | 15.2 ± 1.8 | 22.6 ± 1.8 | 20.0 ± 1.9 |
| 2 | 23.4 ± 1.8 | 17.0 ± 2.0 | 23.6 ± 1.8 | 21.3 ± 1.8 |
| 3 | 24.6 ± 2.3 | 17.8 ± 1.1 | 24.8 ± 1.8 | 21.8 ± 1.6 |
| 4 | 25.4 ± 2.5 | 18.8 ± 1.1 | 24.6 ± 1.8 | 22.0 ± 16 |
| 5 | 26.0 ± 2.3 | 18.2 ± 1.3 | 24.8 ± 1.9 | 21.4 ± 1.1 |

There was an initial depression in food consumption in the DHEA treated mice in the first week. Thereafter the food consumption was equal to or slightly greater than the control mice.

| Compound 2 vs. DHEA | | | | | |
|---|---|---|---|---|---|
| Week | Control | DHEA 0.35% | DHEA 0.7% | Cpd 2 0.35% | Cpd 2 0.7% |
| | | mean weekly weight in grams (n=5) | | | |
| 0 | 21.6 ± 1.3 | 21.1 ± 2.8 | 22.0 ± 2.5 | 21.8 ± 1.6 | 21.3 ± 1.8 |
| 1 | 21.4 ± 2.1 | 20.0 ± 2.3 | 17.0 ± 1.9 | 21.8 ± 1.3 | 20.6 ± 2.3 |
| 2 | 22.0 ± 0.7 | 19.2 ± 1.8 | 17.8 ± 1.8 | 22.0 ± 1.2 | 20.6 ± 0.5 |
| 3 | 22.2 ± 0.8 | 19.6 ± 2.8 | 18.4 ± 1.8 | 22.8 ± 1.3 | 20.8 ± 0.8 |
| 4 | 24.0 ± 1.0 | 22.2 ± 2.4 | 19.2 ± 1.6 | 24.2 ± 1.1 | 22.6 ± 0.6 |
| 5 | 24.8 ± 0.8 | 21.8 ± 2.2 | 20.2 ± 1.9 | 24.4 ± 1.7 | 22.4 ± 0.5 |
| 6 | 25.2 ± 1.1 | 22.8 ± 2.3 | 19.8 ± 2.0 | 24.8 ± 1.5 | 23.5 ± 0.9 |
| 7 | 25.6 ± 1.1 | 23.2 ± 2.4 | 20.6 ± 2.2 | 25.4 ± 1.7 | 23.8 ± 0.8 |

| Compound 16 vs. DHEA | | | | | |
|---|---|---|---|---|---|
| Week | Control | DHEA 0.18% | DHEA 0.35% | Cpd 16 0.18% | Cpd 16 0.35% |
| | | mean weekly weight in grams (n=5) | | | |
| 0 | 21.6 ± 2.8 | 22.4 ± 1.8 | 22.4 ± 2.3 | 22.6 ± 2.7 | 22.0 ± 2.5 |
| 1 | 23.0 ± 1.6 | 20.4 ± 1.9 | 16.0 ± 1.6 | 19.8 ± 2.3 | 16.8 ± 1.6 |
| 2 | 24.4 ± 1.6 | 21.1 ± 0.5 | 20.2 ± 0.9 | 19.4 ± 2.1 | 18.2 ± 0.8 |
| 3 | 25.6 ± 1.9 | 22.4 ± 0.8 | 21.0 ± 0.8 | 19.4 ± 2.1 | 17.2 ± 1.7 |
| 4 | 26.6 ± 1.5 | 23.6 ± 1.5 | 22.2 ± 0.9 | 19.6 ± 2.2 | 17.0 ± 1.2 |

Compound 16 is more than 2X as active as DHEA in this test.

## Anti-Hyperglycemic Activity

Coleman et al. (Diabetes 31 , 830, 1982) reported that administration of DHEA (0.4% of the diet) produced a marked hypoglycemic effect in C57BL/KsJ-db/db mice and significantly prolonged their lifespan. The authors noted that "diabetes is more severe and develops more rapidly in males and can be improved or circumvented by combined estradiol and progesterone treatment" and suggested that the

therapeutic effect of DHEA might result from its metabolism to estrogens. Compound 16 (which has been found herein to be devoid of estrogenic activity in the rat uterotrophic test) was tested in this model.

C57BL/KsJ-db/db 8 week old female mice were obtained and house in polycarbonate cages in animal quarters maintained at 24° C with 12 h of light and 12 h of darkness each day.

Mice were placed on a control chow or a chow diet containing either 0.4% DHEA or 0.2% of compound 16.

For determination of blood glucose levels, mice were bled from the orbital sinus using a heparinized capillary tube. 0.2 ml of blood was added to 1.8 ml of water to hemolyze the blood and glucose concentration was determined using the glucose oxidase assay.

| Blood Glucose Levels (mg/deciliter) | | | |
|---|---|---|---|
| Week | Control (n=6) | DHEA (0.4%) (n=6) | Cpd. 16 (0.2%) (n=6) |
| 0 | 232 ± 43 | 232 ± 28 | 244 ± 32 |
| 1 | 336 ± 37 | 132 ± 14 | 143 ± 21 |
| 2 | 394 ± 20 | 135 ± 37 | 134 ± 13 |
| All mice placed on control diet. | | | |
| 24 hrs. | 399 ± 15 | 192 ± 32 | 147 ± 10 |
| 48 hrs. | 397 ± 13 | 310 ± 37 | 262 ± 23 |

When mice were taken off diets containing DHEA or compound 16 and placed on control diet, blood glucose levels increased at 24 and 48 hrs. but significantly more slowly in the mice that had received compound 16 .

## Anti-Autoimmune Activity

New Zealand Black (NZB) mice develop a progressive autoimmune, Coomb's positive hemolytic anemia with age. It has been previously found that long-term treatment of NZB mice with DHEA significantly inhibits the rate of development of the autoimmune anemia. In other studies reported herein, we have determined that certain steroids, such as compound 16 , have retained the anti-obesity, cancer preventive, and anti-hyperglycemic action of DHEA without any apparent estrogenic effect. There is a reasonable probability that such steroids will also retain the anti-autoimmune activity of DHEA.

The following are additional data on the anti-hyperglycemic effect of compound 16 vs. DHEA.

| Blood Glucose Levels (mg/deciliter) | | | |
|---|---|---|---|
| Week | Control (n=7) | DHEA 0.4% (n=7) | Cpd 16(0.2%) (n=7) |
| 0 | 213 ± 54 | 214 ± 59 | 216 ± 61 |
| 1 | 292 ± 29 | 161 ± 19 | 135 ± 15 |
| 2 | 335 ± 20 | 145 ± 19 | 118 ± 12 |
| 3 | 354 ± 27 | 117 ± 12 | 102 ± 8 |
| 4 | 387 ± 15 | 112 ± 4 | 105 ± 5 |

Compound 16 is more effective in lowering blood glucose concentration at an administered dose of 0.2% in the diet than is DHEA at a dose of 0.4%.

## Anti-Hypercholesterolemic Activity

Six-week old female ICR mice were obtained and placed in animal quarters at 24 °C with 5 animals/cage with food and water ad libitum . All mice (except the control group) received 0.1% PTU (propylthiouracil) in their drinking water. Mice receiving DHEA or compound 4 were injected with the steroid i.p. (15 mg/kg) 3X weekly.

For serum cholesterol determinations, mice were bled from the orbital sinus. Blood was allowed to coagulate and was centrifuged to obtain serum. Cholesterol was measured according to the procedure of Rao et al. (Lipids 12 , 1078, 1977).

| Experimental Group | No. Mice | Serum Cholesterol (mg%) | | |
|---|---|---|---|---|
| | | before treatment | 1wk | 2wk |
| | | | after treatment | |
| Control (no steroid or PTU) | 30 | 53.8 ± 9.3 | 54.0 ± 4.0 | 59.2 ± 7.2 |
| PTU | 40 | 51.3 ± 6.1 | 75.6 ± 12.9 | 76.6 ± 2.4 |
| PTU + DHEA | 10 | 61.0 ± 6.8 | 57.3 ± 3.6 | 57.5 ± 3.2 |
| PTU + Cpd 4 | 10 | 58.1 ± 5.6 | 53.4 ± 5.2 | — |

Uterotrophic Test for Estrogenic Activity. Compound 16 vs. DHEA and Estradiol-benzoate. Compounds Given by Oral Administration.

Twenty-two day old rats were obtained from Charles River Laboratories. Animals were used at 29 days of age. Test steroids were suspended by homogenization in sesame oil. Rats were orally intubated at 1-2 P.M. for 3 days with a test steroid in sesame oil or with sesame oil alone (control). On the 4th day the animals were killed and the uteri were removed and weighed.

| | Mean Uterine Weight (Mgs) (n = 6) |
|---|---|
| Control (no steroid) | 166 ± 34 |
| DHEA (400 mg/kg) | 261 ± 23 ($p < 0.001$, greater than control) |
| Compound 16 (400 mg/kg) | 174 ± 20 |
| Compound 16 (200 mg/kg) | 189 ± 14 |
| Estradiol-benzoate (14 µk/kg) | 244 ± 44 ($p < 0.01$) |

Compound 2 vs. DHEA. Compounds Administered by Subcutaneous Injection

Steroids were dissolved in 2 ml ethanol and brought up to 5 ml volume with propylene glycol. Ccncentrations were such that 1 µl/gm body weight delivered the indicated dose.

| | Mean Uterine Weight (Mgs) (n = 4 or 5) |
|---|---|
| Control (no steroid) | 152 ± 22 |
| DHEA (60 mg/kg) $p < 0.02$ | 290 ± 72 |
| DHEA (10 mg/kg) | 152 ± 17 |
| Compound 2 (60 mg/kg) | 127 ± 32 |
| Compound 2 (10 mg/kg) | 151 ± 32 |

The above test is being repeated at the oral dose of 400 mg/kg, which would have been more appropriate, since this is a therapeutic dose.

Conclusion:

Neither compounds 16 nor 2 are estrogenic at doses at which DHEA is significantly estrogenic.

Summary

Compound 16 is without estrogenic activity in the uterotrophic test and is approximately 3X as active as DHEA in the mouse skin tumor promoter assay and in the anti-obesity test. Compound 2 is without estrogenic activity but is about 1/3 as active as compound 16 in the skin tumor promoter test and only 1/6 as active in the anti-obesity test. However, compound 3 is only about 1/2 as active as compound 16 in the skin tumor promoter test and 1/6 as active in the anti-obesity test.

Compound 15 is about as active as DHEA and compound 16 in the G6PDH inhibition test.

The compounds, i.e. therapeutic agents of this invention may be administered alone or in combination with pharmaceutically-acceptable carriers, the proportion of which is determined by the solubility and chemical nature of the compound, chosen route of administration and standard pharmaceutical practice. For example, they may be administered orally in the form of tablets, pills or capsules containing such excipients as starch, milk sugar, certain types of clay and so forth. They may be administered orally in the form of solutions which may contain coloring and flavoring agents or they may be injected parenterally, that is, intramuscularly, intravenously or subcutaneously. For parenteral administration, they may be used in the form of a sterile solution containing other solutes, for example, enough saline or glucose to make the solution isotonic.

The physician will determine the dosage of the present therapeutic agents which will be most suitable and it will vary with the form of administration and the particular compound chosen, and furthermore, it will vary with the particular patient under treatment. He will generally wish to initiate treatment with small dosages substantially less than the optimum dose of the compound and increase the dosage by small increments until the optimum effect under the circumstances is reached. It will generally be found that when the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

When given orally, the therapeutic doses of the compounds of the present invention are generally in the range of from about 4 to about 450 mg/kg/day depending upon the particular mammalian host and the particular effect desired, e.g. cancer preventive, anti-obesity, anti-diabetes, etc., when given by parenterally, the compounds are administered generally in dosages of, for example, 0.5 to about 15 mg/kg/day also depending upon the host and effect desired.

Obviously, other modifications and variations of the present invention are possible in the light of the above teachings. It is, therefore, to be understood that changes may be made in the particular embodiments of this invention which are within the full intended scope of the invention as defined by the appended claims.

## Claims

1. A compound of the formula:

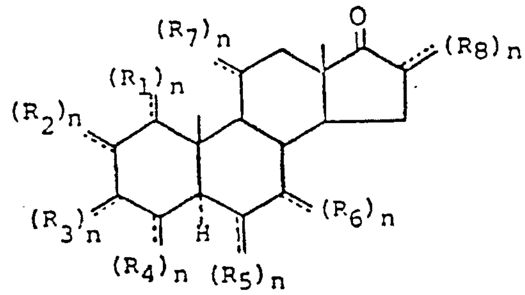

wherein

$R_1$, $R_2$, $R_3$, $R_4$, $R_5$, $R_6$, $R_7$ and $R_8$ are independently selected from the group consisting of hydrogen, alkyl, alkenyl, alkynyl, halogen and hydroxyl, $R_5$ is hydrogen, alkyl, alkenyl, alkynyl or halogen;

n is an integer from 1 to 2 inclusive with the proviso that when $R_1$-$R_8$ are alkenyl or alkynyl, then n is 1 and with the further provisos that $R_3$ may be hydroxy or halogen only when any one of $R_1$, $R_2$, $R_4$, $R_5$, $R_6$, $R_7$ or $R_8$ is other than hydrogen; that said compound is not 4,4-dimethyl-5α, 13α-androstan-17-one; and provided that

when $R_3$ is hydroxy, $R_1$ may be hydroxy or halogen only when any one of $R_2$, $R_4$, $R_5$, $R_6$, $R_7$ or $R_8$ is other than hydrogen;

when $R_3$ is hydroxy, $R_2$ may be methyl or halogen only when any one of $R_4$, $R_5$, $R_6$, $R_7$ or $R_8$ is other than hydrogen;

when $R_3$ is hydroxy, $R_4$ may be halogen, methyl or hydroxy only when any one of $R_1$, $R_2$, $R_3$, $R_5$, $R_6$, $R_7$ or $R_8$ is other than hydrogen;

when $R_3$ is hydroxy, $R_5$ may be methyl, halogen or hydroxy only when $R_1$, $R_2$, $R_4$, $R_6$, $R_7$ or $R_8$ is other than hydrogen;

when $R_3$ is hydroxy, $R_6$ may be hydroxy or methyl only when $R_1$, $R_2$, $R_4$, $R_5$, $R_7$, or $R_8$ is other than hydrogen;

when $R_3$ is hydroxy, $R_7$ may be hydroxy only when $R_1$, $R_2$, $R_4$, $R_5$, $R_6$ or $R_8$ is other than hydrogen;

and when $R_3$ is hydroxy, $R_8$ may be methyl, hydroxy or halogen only when $R_1$, $R_2$, $R_4$; $R_5$, $R_6$ or $R_7$ is other than hydrogen; $R_7$ may be hydroxy only when any one of $R_1$, $R_2$, $R_3$, $R_4$, $R_6$ or $R_8$ is other than hydrogen; and $R_8$ may be bromo only when $R_1$, $R_2$, $R_3$, $R_4$, $R_5$, $R_6$ or $R_7$ is other than hydrogen.

2. The compound according to Claim 1 wherein

$R_4$, $R_6$ and $R_8$ are each independently selected from the group consisting of hydrogen, alkyl, alkenyl, alkynyl, halogen and hydroxy;

$R_1$ and $R_2$ are independently hydrogen, alkyl, alkenyl, alkynyl or halogen;

$R_3$ is hydrogen, alkyl, alkenyl or alkynyl;

$R_5$ is hydrogen, alkyl, alkenyl or alkynyl or halogen;

$R_7$ is hydrogen, alkyl, alkenyl or alkynyl or halogen;

n is an integer from 1 to 2 inclusive, with the provisos that when $R_1$, $R_2$, $R_3$, $R_4$, $R_5$, $R_6$, $R_7$, or $R_8$ is alkenyl or alkynyl, n is 1: that at least one of $R_1$, $R_2$, $R_3$, $R_4$, $R_5$, $R_6$, $R_7$ and $R_8$ is other than hydrogen; that said compound is not 4,4-dimethyl-5α-13α-androstan-17-one; and with the further provisos that

1) when $R_8$ is iodo, chloro or bromo, then at least one of $R_1$-$R_7$ is other than hydrogen;
2) when $R_6$ is fluoro, then at least one of $R_1$, $R_2$, $R_3$, $R_4$, $R_5$, $R_7$ and $R_8$ is other than hydrogen;
3) when $R_5$ is fluoro, then at least one of $R_1$, $R_2$, $R_3$, $R_4$, $R_6$, $R_7$ and $R_8$ is other than hydrogen;
4) when $R_8$ is methyl, than at least one of $R_1$-$R_7$ is other than hydrogen;
5) when $R_8$ is hydroxy, then at least one of $R_1$-$R_7$ is other than hydrogen;
6) when $R_4$ is hydroxy, then at least one of $R_1$, $R_2$, $R_3$, $R_5$, $R_6$, $R_7$ and $R_8$ is other than hydrogen; and
7) when $R_1$ is methyl, then at least one of $R_2$-$R_8$ is other than hydrogen.

3. The compound as in Claims 1 or 2 wherein said alkyl is lower alkyl containing from 1 to 5 carbon atoms.

4. The comound as in any of Claims 1-3 wherein $R_1$, $R_2$, $R_3$ and $R_4$ are each hydrogen or alkyl.

5. The compound as in any of Claims 1-4 wherein $R_3$ is alkyl and $R_1$, $R_2$ and $R_4$ are each hydrogen.

6. A therapeutic composition comprising a therapeutically effective amount of the compound as in any of Claims 1-5 and a carrier therefor.